(19) Europäisches Patentamt
European Patent Office
Office européen des brevets

(11) **EP 3 984 558 A1**

(12) **EUROPEAN PATENT APPLICATION**
published in accordance with Art. 153(4) EPC

(43) Date of publication:
**20.04.2022 Bulletin 2022/16**

(21) Application number: **20813525.1**

(22) Date of filing: **01.06.2020**

(51) International Patent Classification (IPC):
*A61K 48/00* (2006.01)  *A61K 31/713* (2006.01)
*A61K 31/7105* (2006.01)  *A61P 31/20* (2006.01)
*A61P 31/22* (2006.01)  *C12Q 1/70* (2006.01)
*C12N 15/113* (2010.01)

(52) Cooperative Patent Classification (CPC):
**A61K 31/7105; A61K 31/713; A61K 48/00;
A61P 31/20; A61P 31/22; C12N 9/12;
C12N 15/113; C12Q 1/70**

(86) International application number:
**PCT/KR2020/007100**

(87) International publication number:
**WO 2020/242278 (03.12.2020 Gazette 2020/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.05.2019 KR 20190064129**

(71) Applicants:
• **Seoul National University R & DB Foundation
Seoul 08826 (KR)**
• **Institute for Basic Science
Yuseong-gu
Daejeon 34126 (KR)**

(72) Inventors:
• **KIM, V. Narry
Seoul 08826 (KR)**
• **YEO, Jinah
Seoul 08826 (KR)**
• **KIM, Dongwan
Seoul 08826 (KR)**
• **LEE, Young-Suk
Seoul 08826 (KR)**
• **JUNG, Soo-Jin
Seoul 08826 (KR)**

(74) Representative: **J A Kemp LLP
80 Turnmill Street
London EC1M 5QU (GB)**

(54) **METHOD FOR INHIBITING INFECTION AND ACTIVATION OF VIRUS**

(57)    The present invention relates to a technology for preventing or treating virus infection and infectious disease by inducing a mixed tailing regarding an RNA virus

EP 3 984 558 A1

**FIG. 1a**

a   HepG2.2.15

## Description

Technical Field

This disclosure was made with the support of the Ministry of Science and ICT, Republic of Korea, under Project No. 1711079098, which was conducted by the Institute for Basic Science in the research project named "Regulatory RNAs in Cell Fate Decision" under management of the Institute for Basic Science for the project named "Support for research and operation expenses of the Institute of Basic Science", from January 01, 2018 to December 31, 2018.

This application claims priority to and the benefits of Korean Patent Application No. 10-2019-0064129 filed on May 30, 2019 in the Korean Intellectual Property Office, the contents of which are incorporated herein in their entireties by reference.

The present disclosure relates to a technology for preventing or treating viral infections and infection symptoms by inducing mixed tailing to viral RNA.

Background Art

Hepatitis B is a viral disease transmitted by parenteral exposure to infectious matters such as blood, blood products, contaminated needles, etc., by sexual contact with infected carriers, and by vertical transmission from mother to child during childbirth. According to the World Health Organization, more than 2 billion people have been infected worldwide while about 4 million cases of acute hepatitis B occurred each year, with one million people dying every year due to the infection. An estimated 350-400 million people are chronic media (World Health Organization: Geographic Prevalence of Hepatitis B Prevalence, 2004. http://www.who.int/vaccines-surveillance/graphics/htmls/hepbprev.htm).

Hepatitis B virus (HBV) is hepatotropic virus with double-stranded DNA and infects only humans and nonhuman primates. Viral replication occurs mainly in the liver, and to lesser extents in the kidney, pancreas, bone marrow, and spleen (Hepatitis B virus biology. Microbiol Mol Biol Rev. 64: 2000; 51-68.). Viral and immune markers can be detected in blood, and characteristic antigen-antibody patterns evolve with time. The first detectable viral marker is HBsAg, followed by hepatitis B e antigen (HBeAg) and HBV DNA. During the incubation period, the titer may be high, but HBV DNA and HBeAg levels begin to decline at the onset of the disease and may be undetectable at the clinical peak of the disease (Hepatitis B virus infection natural history and clinical consequences. N Engl J Med. 350: 2004; 1118-1129). HBeAg is a viral marker detectable in blood and is associated with active viral replication, and thus, the viral load and infectivity are high (Hepatitis B virus e antigen the dangerous end game of hepatitis B virus. N Engl J Med. 347: 2002; 208-210). The presence of anti-HBsAb and anti-HBcAb (IgG) accounts for the recovery and immunity in already infected individuals.

At present, for adults with chronic HBV infection, the American Association for the Study of Liver Diseases (AASLD) and the European Association for the Study of the Liver (EASL) recommend as the first-line therapy interferon alpha (IFNα), pegylated interferon alpha-2a (Peg-IFN2a), entecavir, and tenofovir. Nucleoside and nucleotide therapies, entecavir, and tenofovir succeed in reducing viral loads, but exhibit seroconversion and HBsAg loss at much lower levels, compared to IFNα therapy. Other similar therapies including lamivudine

(3TC), telbivudine (LdT), and adefovir are also utilized, but as for nucleoside/nucleotide therapies, their therapeutic effects are generally limited by the resistance generation.

Therefore, there is a need for finding and developing a novel anti-viral therapy in the field.

Human cytomegalovirus, a member of the viral family Herpesviridae, is a widespread pathogen. The virus consists of a capsid containing linear double-stranded deoxyribonucleic acid (DNA), surrounded by tegument and enclosed by envelop consisting of a lipid bilayer with glycoproteins spiked thereon. Like other members of the family, HCMV possesses the feature of latency and reactivation. HCMV has the ability to infect many cells and remain latent therein.

In immunocompetent hosts, most HCMV infections are asymptomatic or very mild with a little non-specific symptom, for example, slight increases in fatigue, boredom, moderate fever, lymphadenopathy, hepatomegaly, or liver enzymes. However, heterophile-negative mononucleosis is observed in approximately 10% of individuals healthy previously.

For the therapy of HCMV, a new strategy is also required.

Disclosure of Invention

Technical Problem

Leading to the present disclosure, inventive and thorough research conducted by the present inventors resulted in the finding that TENT4A/B are responsible for mixed tailing of viral RNA, more specifically, mRNA and are helpful for inhibiting mRNA degradation in host cells and that downregulation of the expression and activity of TENT4A/B in host cells of a subject inhibits the mixed tailing to promote the deadenylation of viral RNA, with the consequent rapid degradation of viral RNA.

Therefore, an aspect of the present disclosure is to provide a pharmaceutical composition for prevention of viral infection or treatment of viral infection symptoms.

Another aspect of the present disclosure is to provide a method for screening a pharmaceutical composition for prevention of viral infection or treatment of viral infection symptoms.

Another aspect of the present disclosure is to provide a method for preparation of a virus-resistant cell.

Another aspect of the present disclosure is to provide a method for stabilization of RNA sequences.

Solution to Problem

According to an aspect thereof, the present disclosure provides a pharmaceutical composition for prevention of viral infection or treatment of viral infection symptoms, the composition comprising an inhibitor against at least one of TENT4A and TENT4B, both responsible for mixed tailing of viral RNA, wherein the inhibitor is siRNA or shRNA inhibiting expression of at least one of TENT4A and TENT4B, or an antibody or antigen binding fragment thereof inhibiting activity of at least one of TENT4A and TENT4B.

The present inventors discovered that TENT4A/B are involved in mixed tailing of viral RNA, especially mRNA, to help inhibit the degradation of mRNA in host cells and found that downregulation of the expression and activity of TENT4A/B in host cells of a subject inhibits the mixed tailing to promote the deadenylation of viral RNA, with the consequent rapid degradation of viral RNA.

RNA tailing (non-templated nucleotide addition to the 3' end) is the most widespread and conserved type of RNA modification. Noncanonical poly(A) polymerases, also known as terminal nucleotidyltransferases (TENTs), posttranscriptionally incorporate nucleotides to the 3' end of RNAs to regulate their maturation, stability and activity. Previously, the present inventors developed a method called TAIL-seq to explore the RNA 3' extremity and measure the poly(A) tail length. From TAIL-seq experiments, noncanonical tailing events such as uridylation and guanylation on vertebrate mRNAs were found. Terminal uridylyltransferases (TUT4 and TUT7) uridylate deadenylated poly(A) tails (shorter than ~25 nucleotides) after PABPC is removed. U tails, especially oligo-U tails, facilitate mRNA decay. In contrast, guanylation occurs on long poly(A) tails and protects mRNAs from rapid deadenylation. Two related enzymes TENT4A (also known as PAPD7 or TUT5) and TENT4B (also known as PAPD5 or TUT3) extend mRNA poly(A) tails with intermittent nonadenosine residues (most commonly guanosine) to generate "mixed tails". This non-pure poly(A) tail impedes deadenylation by the CCR4-NOT (CNOT) complex and enhances the stability of the mRNA. Mixed tailing was proposed to serve as a new type of post-transcriptional regulation.

As used herein, the term "mixed tailing" refers to substitution of guanine or uridine for at least one residue on the poly(A) tail of viral RNA, more specifically viral mRNA by guanylation or uridylation. Herein, the term "mixed tailing" may be simply written as "mix tailing", "tailing", etc. When guanylation or uridylation occurs thereon, the poly(A) tail sequence of viral RNA is protected from deadenylation, with the resultant delay of mRNA decay.

It was first discovered in the present disclosure that TENT4A and TENT4B, which are noncanonical poly(A) polymerases belonging to a family of terminal nucleotidyltransferases, are involved in mixed tailing of the poly(A) tail of viral mRNA.

In an embodiment of the present disclosure, TENT4A and TENT4B of the present disclosure is derived from a subject which is at risk of viral infection or has been infected with virus. The present inventors investigated that after infecting a subject, the viral mRNA can be induced to undergo mixed tailing by the TENT4A and TENT4B derived from the subject.

In an embodiment of the present disclosure, the virus of the present disclosure is a virus which undergoes the RNA tailing induced by at least one of TENT4A and TENT4B. In greater detail, the present inventors founds that the post-transcriptional regulatory element (PRE), specifically the CNGGN-type pentaloop, is critical for TENT4A/B-dependent regulation. In addition, the present inventors revealed that both HBV and HCMV act in similar manners of using pentaloop to recruit TENT4A/B which in turn induces mixed tailing. The two distinct viruses HBV and HCMV (one belonging to Hepadnaviridae and the other to Herpesviridae) with very different life cycles and tissue specificity employ an identical strategy for the benefit of their gene expression, and this convergent evolution and the simplicity of this cis-acting element intimate that this mechanism may be used by other viruses as well.

In an embodiment of the present disclosure, the virus of the present disclosure is a virus belonging to the family Hepadnaviridae or Herpesviridae.

In an embodiment of the present disclosure, the virus belonging to the family Hepadnaviridae according to the present disclosure is any one selected from the group consisting of hepatitis B virus, ground squirrel hepatitis B virus, woodchuck hepatitis B virus, duck hepatitis B virus, and heron hepatitis B virus.

In an embodiment of the present disclosure, the virus belonging to the family Herpesviridae is any one selected from the group consisting of Iltovirus, Mardivirus, Scutavirus, Simplexvirus, Varicellovirus, Cytomegalovirus, Muromegalovirus, Proboscivirus, Roseolovirus, Lymphocryptovirus, Macavirus, Percavirus, and Rhadinovirus.

As used herein, the term "siRNA" refers to a short double-stranded RNA capable of inducing the RNAi (RNA interference) phenomenon by cleaving a specific mRNA. It may consist of a sense RNA strand having a sequence homologous to the mRNA of a target gene and an antisense RNA strand having a sequence complementary thereto. Because the siRNA can inhibit the expression of the target gene, it can be provided as an effective tool for gene knockdown or gene therapy.

The double-stranded RNA portions of siRNAs in which two RNA strands pair up are not limited to the completely paired ones, and may contain nonpairing portions due to mismatch (the corresponding nucleotides are not complementary), bulge (lacking in the corresponding complementary nucleotide on one strand), and the like. siRNA is 10 to 100 base pairs, particularly 15 to 80 base pairs, and more particularly 20 to 70 base pairs in total length. The terminal structure of siRNA may be either blunt or cohesive as long as siRNA enables to silence the target gene expression due to its RNAi effect. As the cohesive terminal structure, both 3' overhang and 5' overhang structures are possible, with no limitations imparted to the number of overhang nucleotides. In addition, so long as siRNA is able to maintain its gene silencing effect on the target gene, siRNA may contain a low molecular weight RNA (which may be a natural RNA molecule such as tRNA, rRNA or viral RNA, or an artificial RNA molecule), for example, in the overhanging portion at its one end. The terminal structure of the siRNA is not necessarily a cut off structure at both ends and may have a stem-loop structure in which ends of one side of double-stranded RNA are connected by a linker RNA.

The siRNA may be a complete form having a polynucleotide pairing, i.e., a form in which siRNA is synthesized directly in vitro and then introduced into cells through a transformation process, or a form in which a single-chain polynucleotide and its reverse complement can be induced from a single-chain oligonucleotide fragment separated by a spacer after being administered in vivo, for example, a form in which a siRNA expression vector or a PCR-induced siRNA expression cassette is transformed or transfected into cells so that the siRNA is expressed in the cells. The determination of methods for preparing siRNA and introducing siRNA into cells or animals may be dependent on cell biological functions of object or target gene products.

The term "shRNA", as used herein, is intended to overcome the disadvantages of the siRNA, i.e., high cost of biosynthesis, short-term maintenance of RNA interference effect due to low cell transfection efficiency, etc. It may be expressed after being introduced into cells from a promoter of RNA polymerase III using an adenovirus, lentivirus or plasmid expression vector system. The shRNA may induce silencing of a target gene after being converted to a siRNA with an accurate structure by a siRNA-processing enzyme (Dicer or RNase III) existing in cells.

In an embodiment of the present disclosure, the siRNA of the present disclosure is exemplarily given in Table 1, but is not limited thereto.

The antibody of the present disclosure, which inhibits the activity of at least one of TENT4A and TENT4B, is intended to encompass a monoclonal antibody and a chimeric antibody thereof, a humanized antibody, and a human antibody and may include antibodies already known in the art as well as novel antibodies. The antibody may be in an intact full-length antibody having two heavy chains and two light chains or a functional fragment of the antibody molecule, as long as it binds specifically to TENT4A and/or TENT4B. The functional fragment of the antibody molecule refers to a fragment possessing at least an antigen-binding function, and may be Fab, F(ab'), F(ab')2, or Fv.

In the following Examples of the present disclosure, the following antibodies were employed, but with no limitations thereto:

Anti-TENT4A antibody (Invitrogen, PA5-61302), anti-TENT4A antibody (Atlas Antibodies, HPA045487), anti-TENT4A mouse antibody (labmade), anti-TENT4B antibody (invitrogen, PA5-60177), anti-TENT4B antibody (Atlas Antibodies, HPA042968), anti-TENT4B mouse antibody (labmade).

When the composition of the present disclosure is used as a pharmaceutical composition, the content of the active ingredient in the composition may be appropriately adjusted depending on symptoms of the disease, progression of the symptoms, conditions of the patient, etc. For example, it may be contained in an amount of 0.0001 to 99.9 % by weight, based on the total weight of the composition, but with no limitations thereto. The content ratio is on the basis of the dry mass resulting from solvent removal.

The pharmaceutical composition may contain a suitable carrier, excipient, and diluent typically used for preparing adequate administration formulations. According to typical methods, the pharmaceutical composition may be prepared into a formulation for oral administration such as a powder, a granule, a tablet, a capsule, a suspension, an emulsion, a syrup, an aerosol, etc., a formulation for external use, a suppository or a sterilized injectable solution.

For formulating the pharmaceutical composition, typical diluents or excipients such as fillers, thickeners, binders, humectants, disintegrants, surfactants, etc. may be employed. Solid formulations for oral administration include a tablet, a pill, a pulvis, a granule, a capsule, etc. These solid formulations may contain at least one excipient and/or lubricant. Liquid formulations for oral administration include a suspension, a solution for internal use, an emulsion, a syrup, etc. In addition to a simple diluent such as water and liquid paraffin, various excipients, e.g., a humectant, a sweetener, an aromatic, a preservative, etc. may be included. As formulations for parenteral administration, a sterilized aqueous solution, a non-aqueous solution, a suspension, an emulsion, a lyophilized formulation or a suppository may be used.

The preferable dose of the pharmaceutical composition may vary depending on the state and body weight of the patient, the severity of disease, drug forms, administration routes and duration and may be suitably selected by a person skilled in the art. For more preferable effects, the composition of the present disclosure may be administered at a dose of 0.1 mg/kg to 100 mg/kg a day for the active ingredient, but with no limitations thereto. Administration may be conducted once a day or in divided doses multiple times a day. The composition of the present disclosure may be administered to mammals including humans through various routes. All modes of administration can be contemplated, for example, oral administration, intravenous, intramuscular, subcutaneous injections, etc. may be taken. For a pharmaceutical dosage form thereof, the composition according to the present disclosure may be accounted for entirely by the form of a pharmaceutically acceptable salt of the active ingredient. The composition may be used alone or in combination with another pharmaceutically active compound or in a suitable cluster.

Another aspect of the present disclosure provides a method for screening a pharmaceutical composition for prevention of viral infection or treatment of viral infection symptoms, the method comprising the steps of:

(a) transfecting a virus into host cells;
(b) treating the host cells with a drug candidate material; and
(c) analyzing an RNA tailing level of a test group treated with the drug candidate material and comparing the RNA tailing level with that of a control treated without the drug candidate material, wherein the drug candidate material is selected as an effective candidate when the RNA tailing level of the test group is relatively reduced compared to the non-treated control.

The RNA tailing level is determined in terms of the number of substitution of other nucleosides such as guanines or uridines for adenines on the poly(A) tail through guanylation or uridylation.

In the present disclosure, steps (a) and (b) may be conducted in the forward or reverse order.

When the mixed tailing in virus-infected host cells is inhibited by treatment with a sample, selection may be made of the sample as an effective drug candidate material.

The drug screening method of the present disclosure utilizes the mixed tailing operation by TENT4A/B in common with pharmaceutical composition according to another aspect of the present disclosure and invokes the description of the pharmaceutical composition, and the description of overlapping contents therebetween will be omitted to avoid excessive complexity of the present specification.

According to another aspect thereof, the present disclosure provides a method for preparing a virus-resistant cell, the method comprising a step of knocking out expression of at least one of TANT4A and TENT4B in a cell isolated from a living body.

For the knockout, various genome editing techniques can be carried out as described previously. In the Examples of the present specification, CRISPR-Cas genome editing was utilized (see Example 6), but without limitations thereto.

According to another aspect thereof, the present disclosure provides a method for stabilizing an RNA sequence, the method comprising a step of inserting a stem-loop sequence including a pentaloop structure consisting of the amino acid sequence represented by the following general formula into a target RNA sequence:

[General Formula]

5'-CNGGN-3',
wherein N's are each independently selected from adenosine (A) and uracil (U).

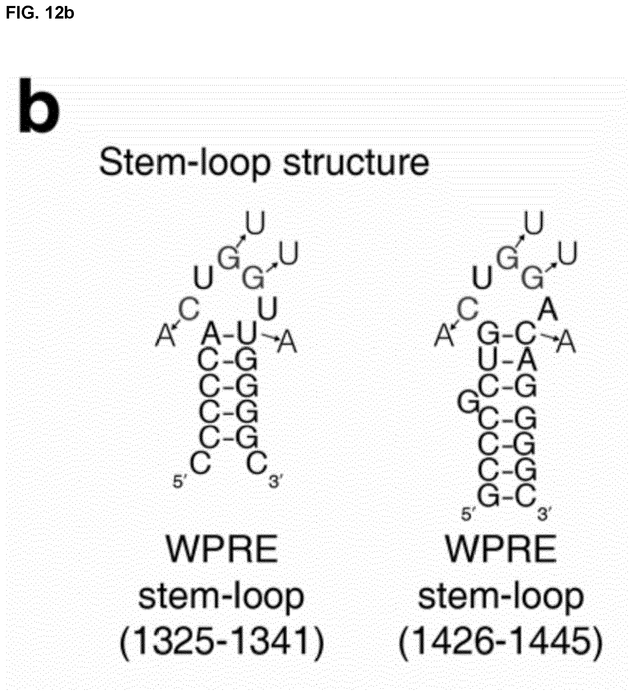

As used herein, the term "stem-loop structure" is an intramolecular base pairing that can occur in single stranded DNA or RNA if sequences of two regions of the same strand are complementary to each other. This structure is also known as a hairpin or hairpin structure. Examples of stem-loop structures are illustrated in FIG. 12B, but are not limited thereto.

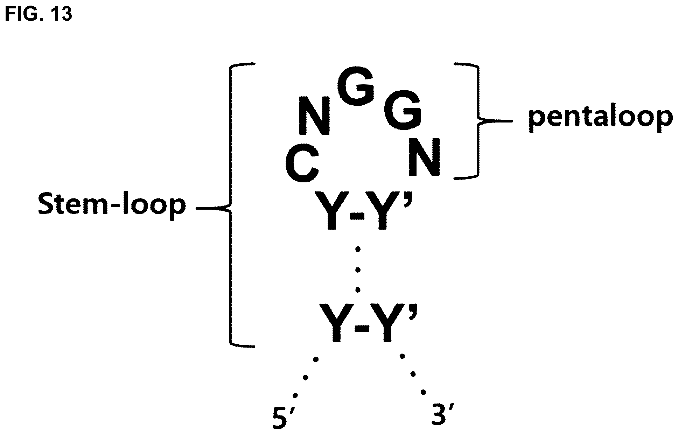

As used herein, the term "pentaloop" refers to a single-stranded sequence region of 5 bases located at a linker that has no base pairing interactions on a stem-loop structure. The stem-loop structure of the present disclosure includes a pentaloop structure as illustrated in FIG. 13.

In an embodiment of the present disclosure, the stem-loop structure of the present disclosure includes 2-15 base pair bonds, more specifically, for example, 2-14 base pair bonds, 2-13 base pair bonds, 2-12 base pair bonds, 2-11 base pair bonds, 2-10 base pair bonds, 3-10 base pair bonds, and 3-9 base pair bonds, but without limitations thereto. The base pair bonds may be adenineuracil bonds or cytosine-guanine bonds, but without limitations thereto.

Insertion of the stem-loop structure of the present disclosure into a target RNA sequence increases the frequency of inducing mixed tailing with TENT4A/B in the target RNA sequence, resulting in stabilizing the RNA sequence.

An exemplary embodiment of the present disclosure is illustrated in FIG. 13.

Advantageous Effects of Invention

Features and advantages of the present disclosure are as follows:

(a) The present disclosure provides a pharmaceutical composition for prevention of viral infection and treatment of viral infection symptoms.
(b) The present disclosure provides a method for screening a pharmaceutical composition for prevention of viral infection and treatment of viral infection symptoms.
(c) The present disclosure provides a method for preparing a virus-resistant cell.
(d) The present disclosure provides a method for stabilizing an RNA sequence by incorporating a predetermined stem-loop structural sequence.
(e) When used, the pharmaceutical composition of the present disclosure can effectively prevent and treat viral infection.
(f) The RNA sequence stabilizing method of the present disclosure can induce mixed tailing in a desired RNA sequence to stabilize the sequence.

Brief Description of Drawings

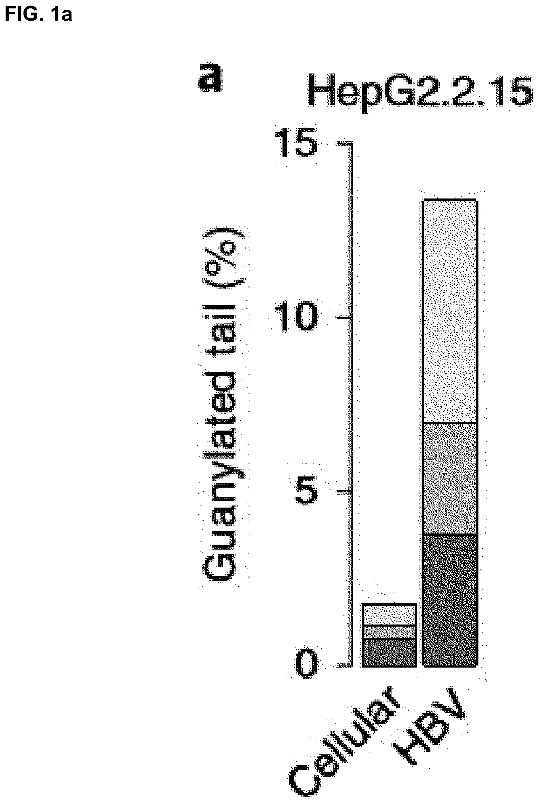

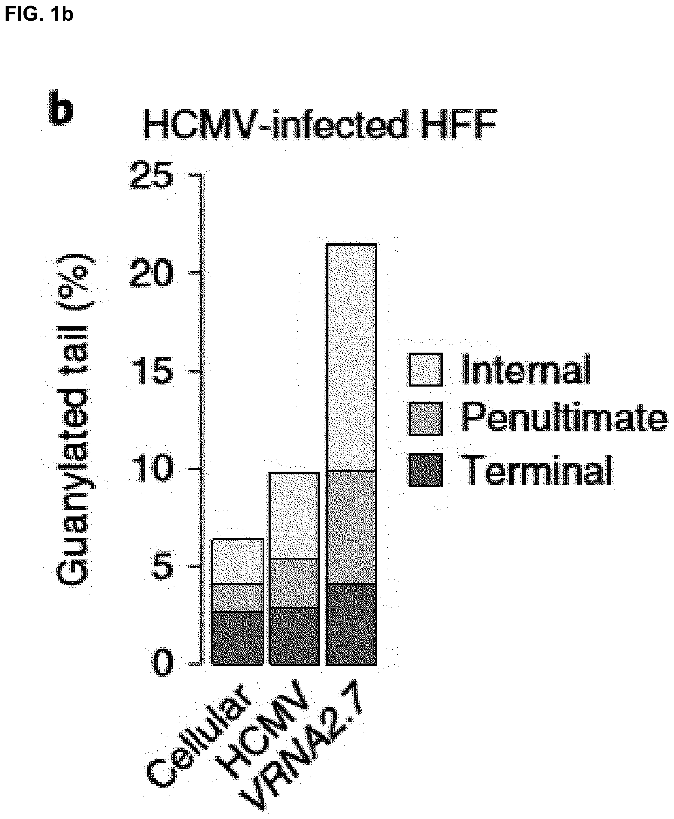

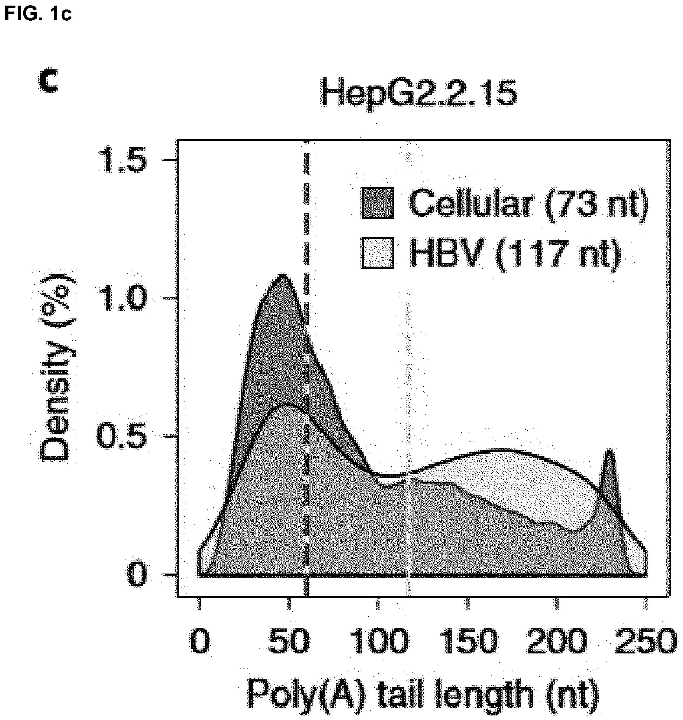

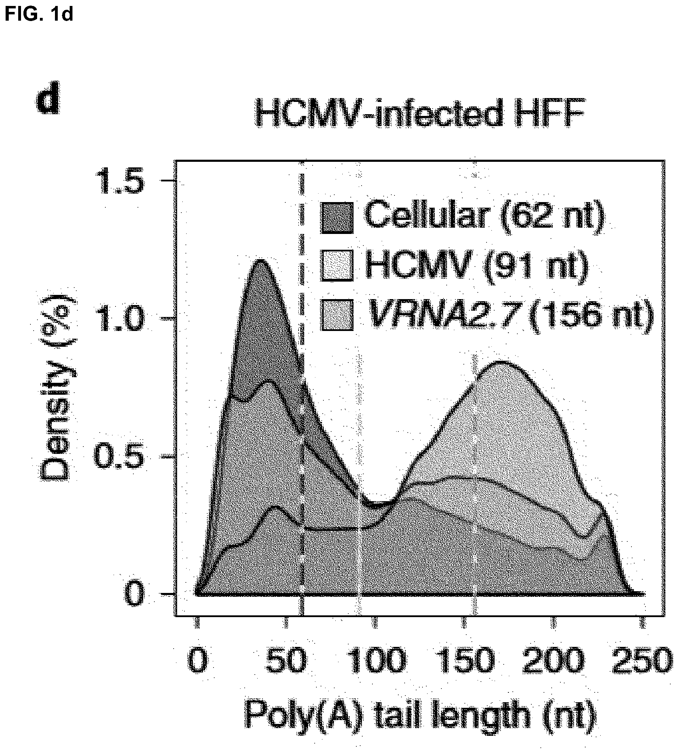

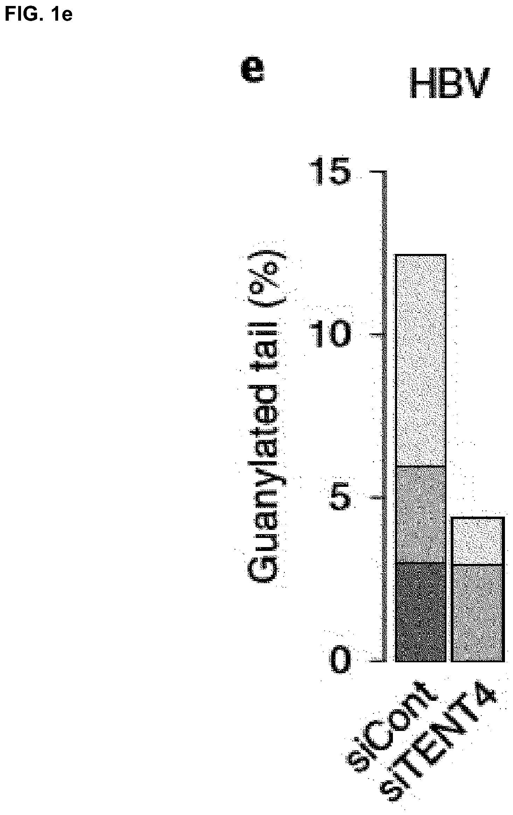

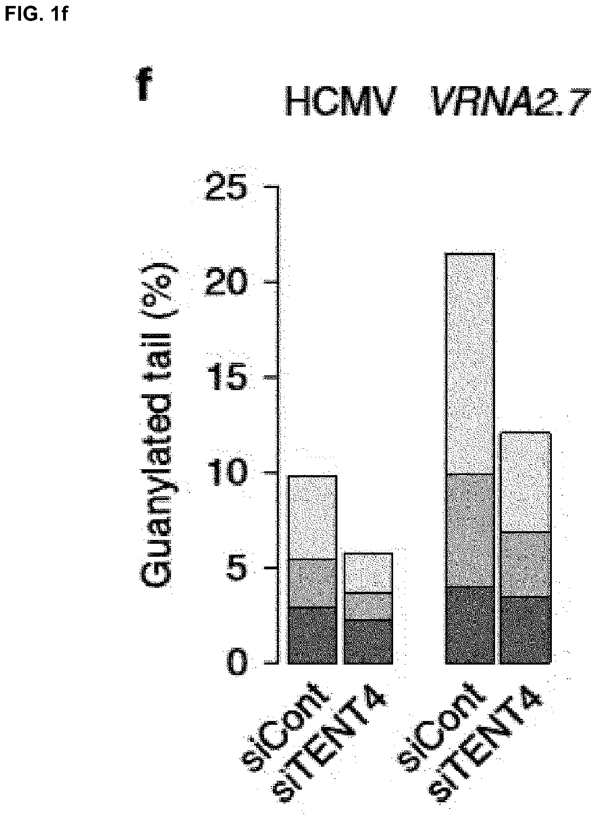

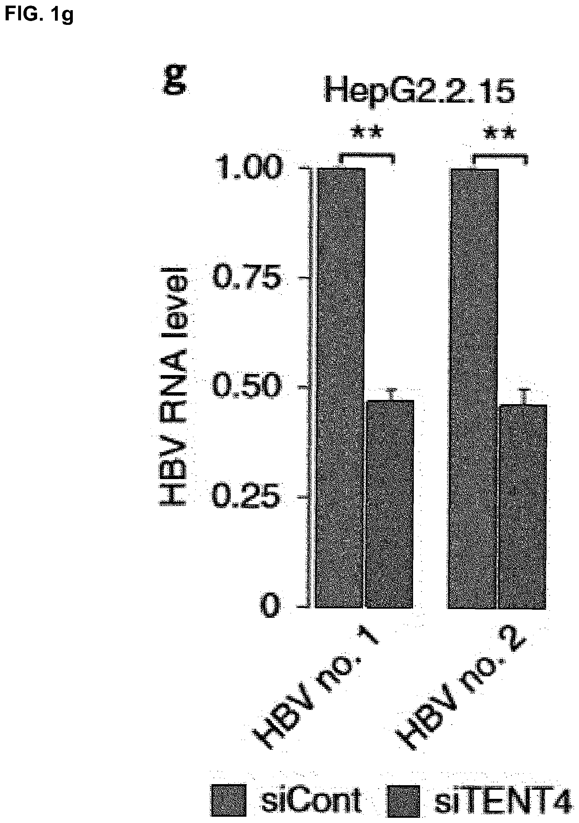

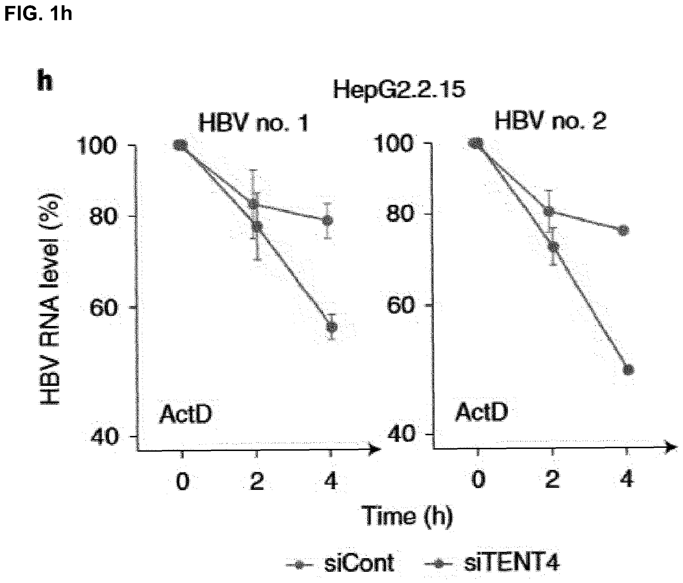

FIG. 1 shows extensive mixed tailing of viral RNAs. a, The fraction of guanylated tails of cellular and viral mRNAs was calculated for mRNAs with a poly(A) tail length of 25 nt or more (n=2 TAIL-seq experiments). b, The fraction of guanylated tails of cellular RNAs, HCMV RNA and RNA2.7 (VRNA2.7) was calculated for RNAs with a poly (A) tail length of 25 nt or more (n=1 TAIL-seq experiments). c, Global poly(A) tail length distributions of cellular (gray) and HBV (yellow) mRNAs. Medians are marked by dashed vertical lines and the median length is shown in parentheses. d, Global poly(A) tail length distributions of cellular (gray), HCMV (yellow) and VRNA2.7 (orange) RNAs. Medians are marked by dashed vertical lines and the median length is shown in parentheses. e, Fraction of guanylated tails of HBV mRNAs with a poly(A) tail length of 25 nt or more in TENT4-depleted HepG2.2.15 cells (n = 1 TAIL-seq experiment). f, Fraction of guanylated tails of HCMV RNAs with a poly(A) tail length of 25 nt or more in TENT4-depleted, HCMV-infected HFF cells (n=1 TAIL-seq experiment). g, HBV mRNA levels in TENT4-depleted HepG2.2.15 cells were measured by RT-qPCR using two separate primer sets. Data are represented as mean±s.e.m. (n= 3 independent experiments). \*\*P<0.01; two-sided Student's t-test. h, Half-lives of HBV mRNAs were measured by RT-qPCR using two separate primer sets. Data are represented as mean±s.e.m. (n= 3 independent experiments). GAPDH mRNA was used for normalization.

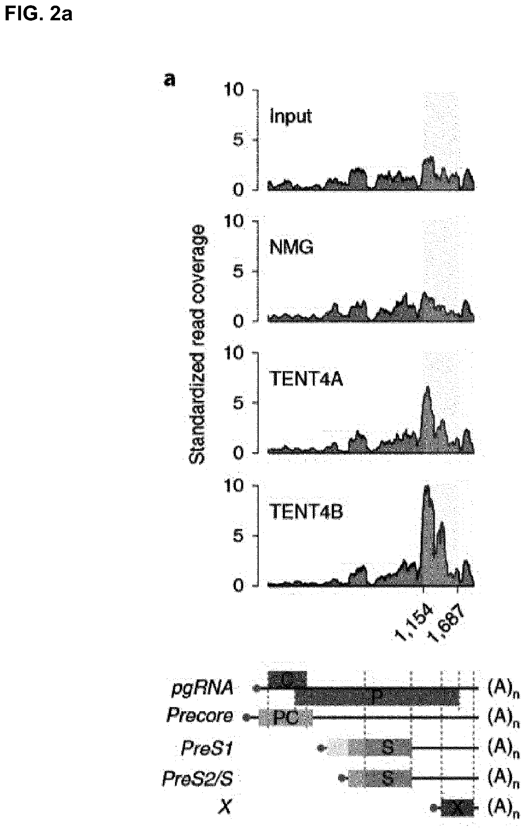

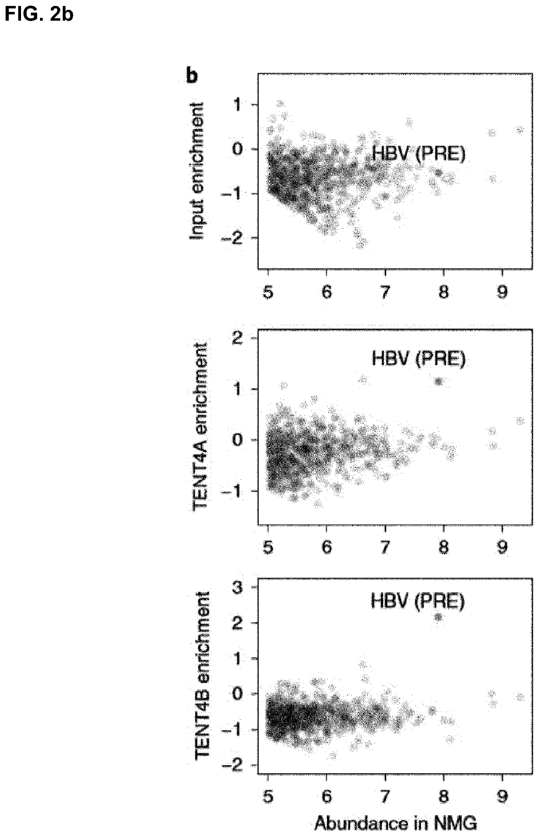

FIG. 2 shows data obtained from experiments demonstrating that HBV RNAs are the major mRNA substrates of TENT4 via the PRE of HBV. a, Standardized read coverage of fCLIP-seq libraries across the HBV genome (NCBI U95551.1). The PRE region of HBV is highlighted in yellow. The HBV transcripts and their respective coding sequence regions are shown below as a reference. Input and NMG libraries were used as negative controls. b, Scatter plot of abundance (x axis) and enrichment score (y axis) of fCLIP-seq peak clusters. HBV PRE is shown as a red dot. A size-matched input library was used as a negative control for this analysis.

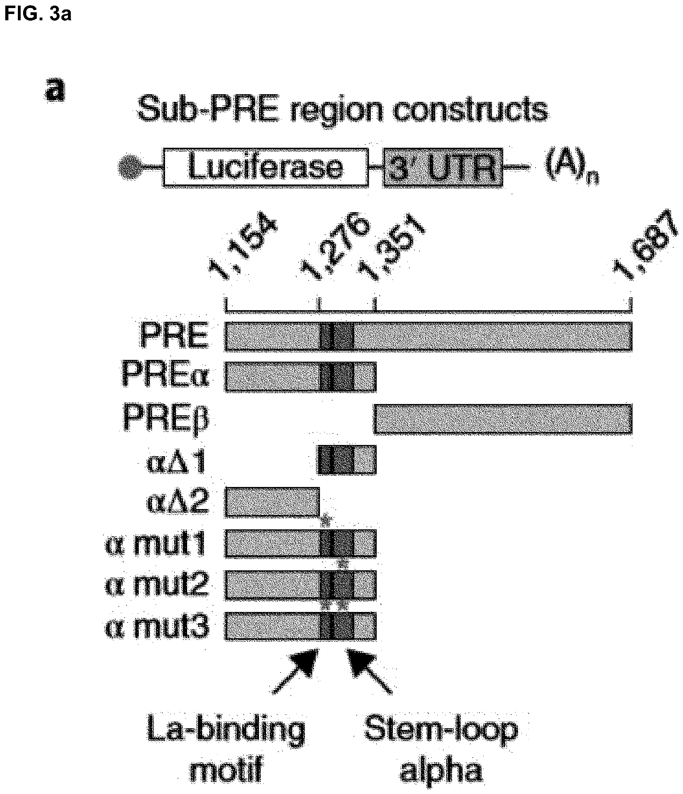

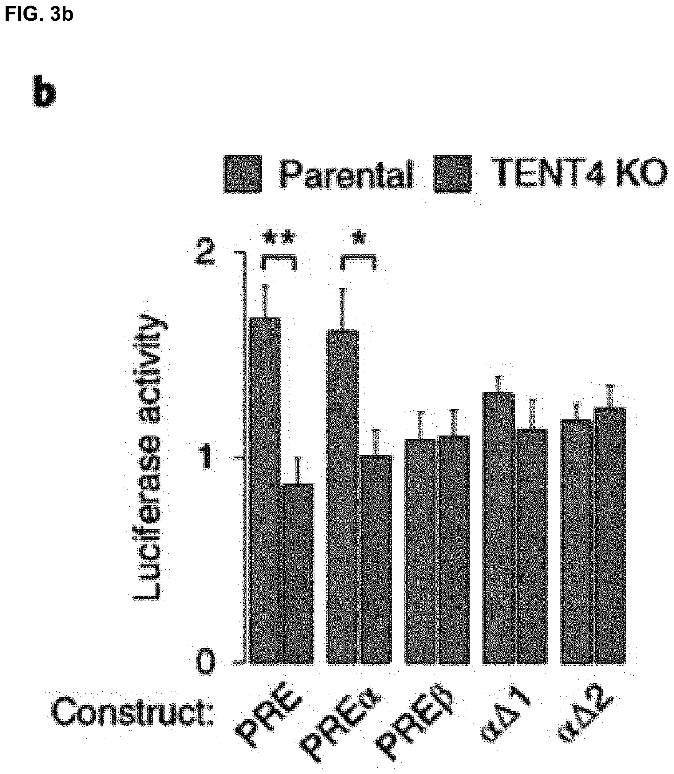

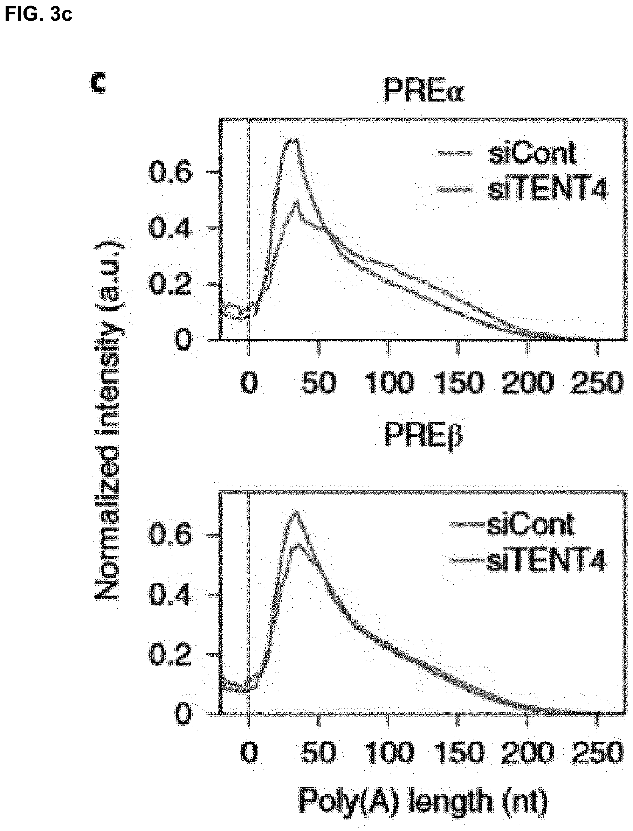

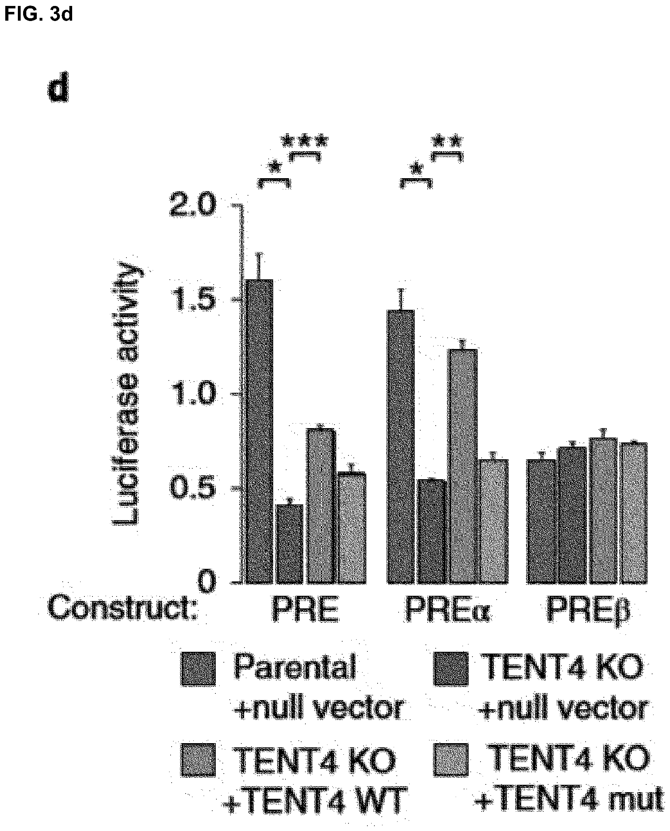

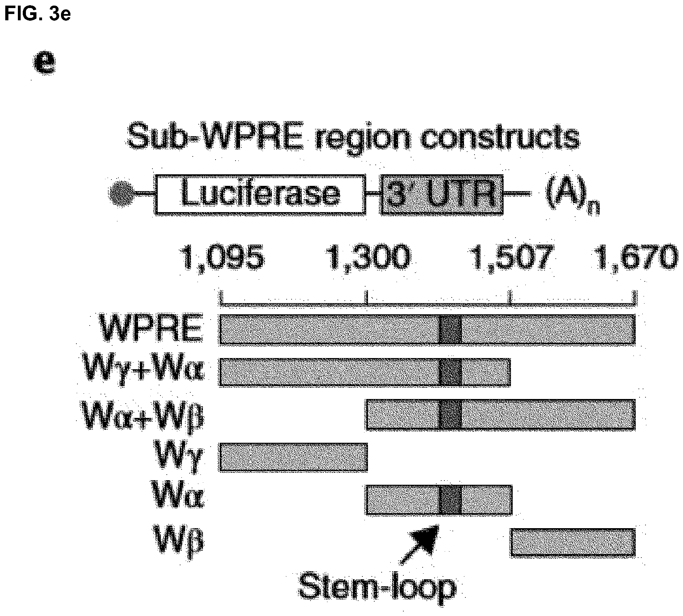

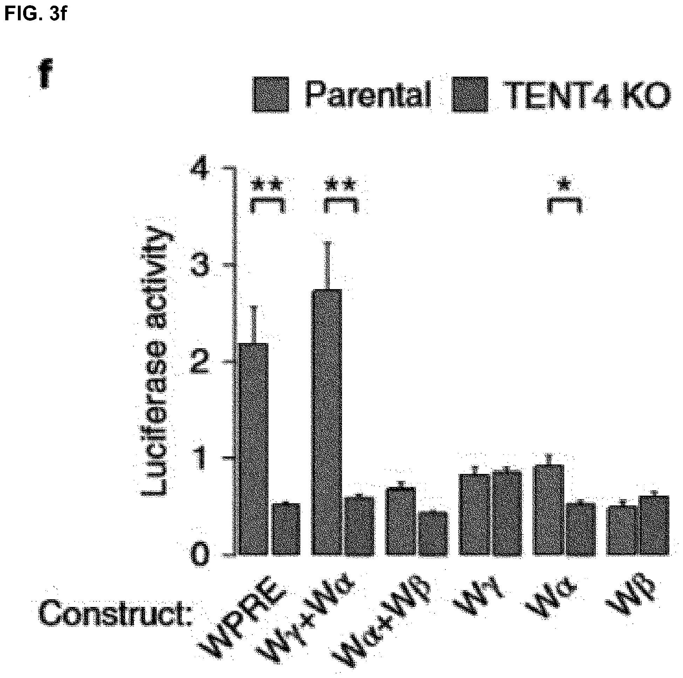

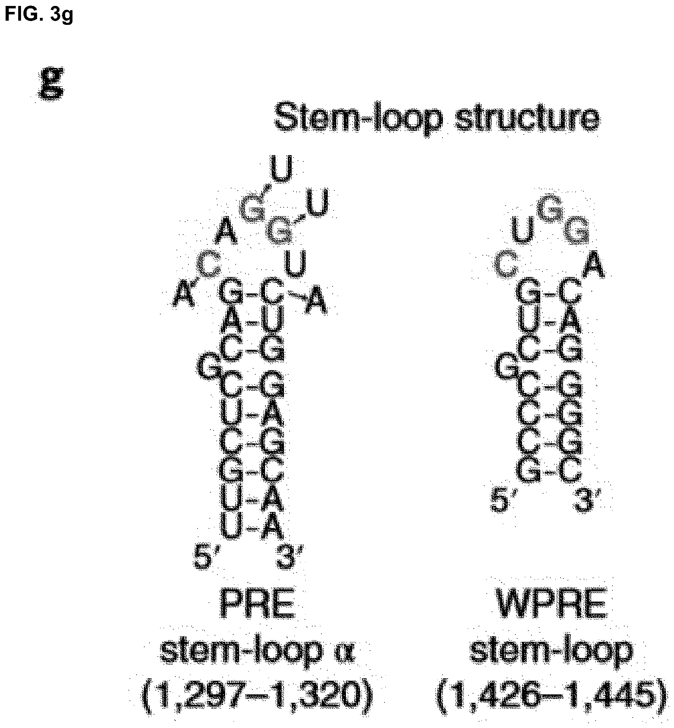

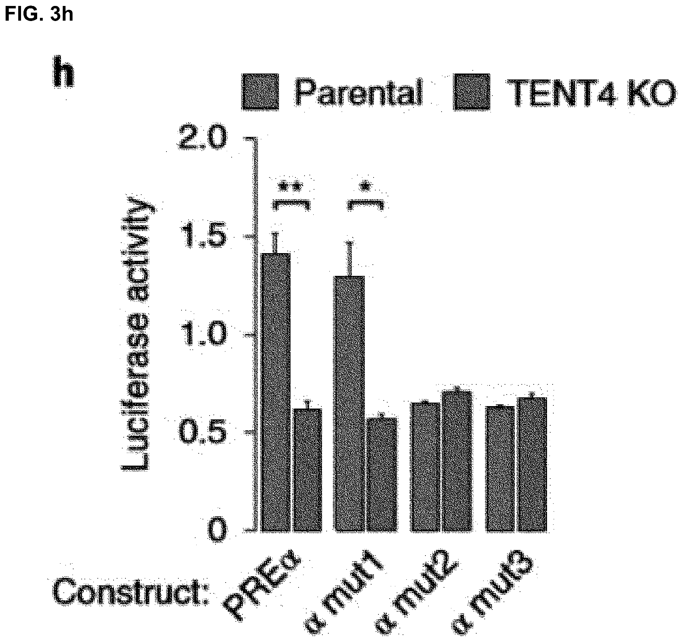

FIG. 3 shows that the stem-loop structure of PRE is necessary but may not be sufficient for TENT4-dependent RNA tailing of HBV mRNA. a, Schematic view of firefly luciferase reporter constructs harboring subregions and mutants of PRE in their 3' UTRs. Blue, La-binding motif; green, stem-loop alpha; and red asterisk, region of point mutation. b, Firefly luciferase activity of reporters containing subregions of PRE in parental HeLaT (gray) and TENT4 KO (red) cells. Data are represented as mean±s.e.m. (n=7 independent experiments for PRE, PREα and PPEβ, n=6 independent experiments for all others). Luciferase activities of both renilla and control vector were used for normalization. \*P<0.01, \*\*P<0.001; two-sided t-test. c, Distributions of poly(A) tail length of reporter RNAs measured by Hire-PAT assay. The reporter constructs containing PREα and PREβ sequences were transfected into HEK293T cells after TENT4 depletion. d, Firefly luciferase activity of PRE reporter constructs in parental or TENT4 KO cells with ectopically expressed wild-type TENT4 or a catalytically inactive mutant. Data are represented as mean±s.e.m. (n=3 independent experiments). Both renilla and control vector were used for normalization. \*P<0.05, \*\*P<0.01, \*\*\*P<0.001; two-sided Student's t-test. e, Schematic diagram of firefly luciferase reporter constructs harboring subregions of WPRE in their 3' UTRs. f, Firefly luciferase activity of WPRE reporter constructs in TENT4 KO cells. Data are represented as mean±s.e.m. (n=3 independent experiments for Wβ, n=7 independent experiments for all others). Luciferase activities of both renilla and control vector were used for normalization. \*P<0.05, \*\*P<0.01; two-sided Student's t-test. g, Stem-loop structures of HBV PRE, mutant HBV PRE and WPRE. h, Firefly luciferase activity of mutant PRE reporter constructs shown in a in TENT4 KO cells. Data are represented as mean±s.e.m. (n=4 independent experiments). Luciferase activities of both renilla and control vector were used for normalization. \*P<0.05, \*\*P<0.01; two-sided Student's t-test.

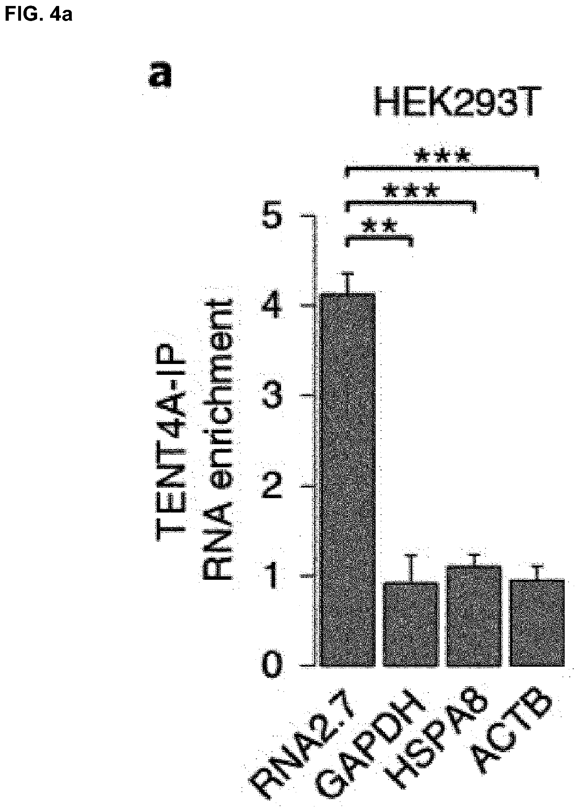

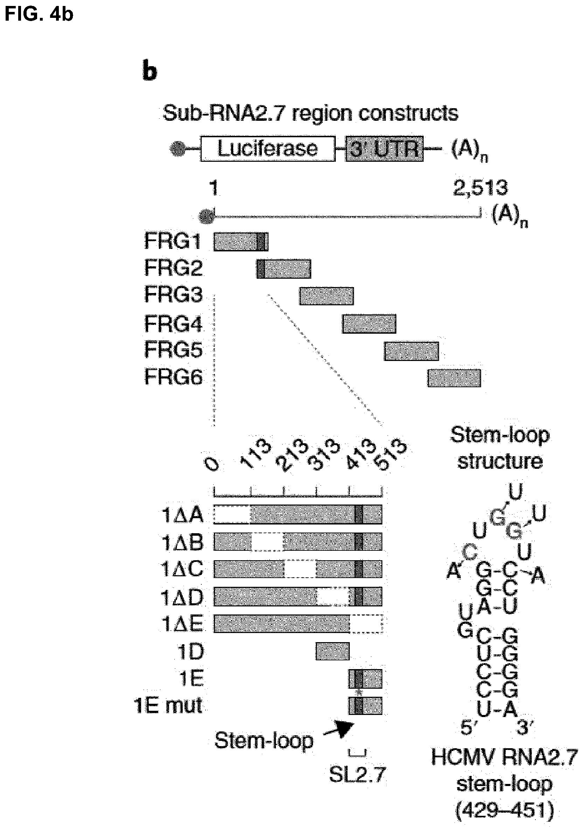

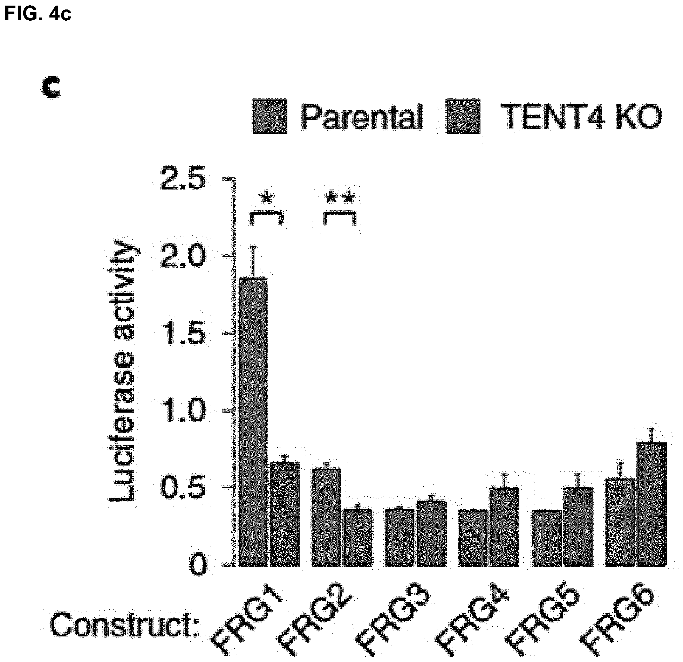

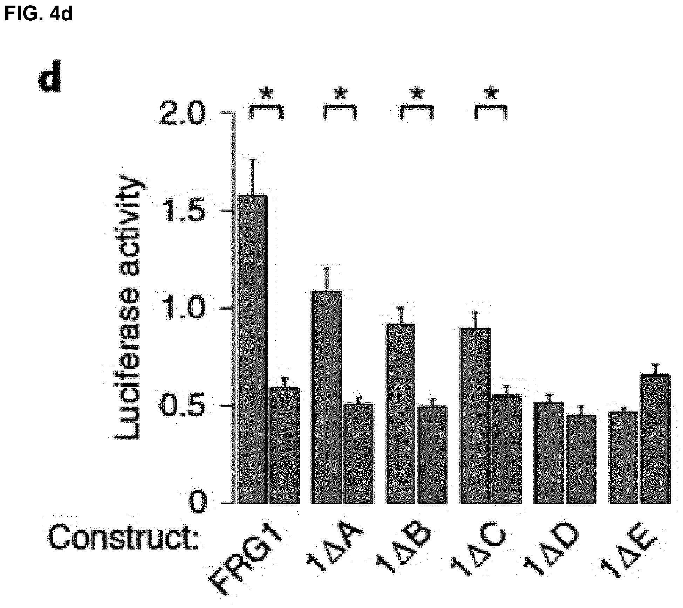

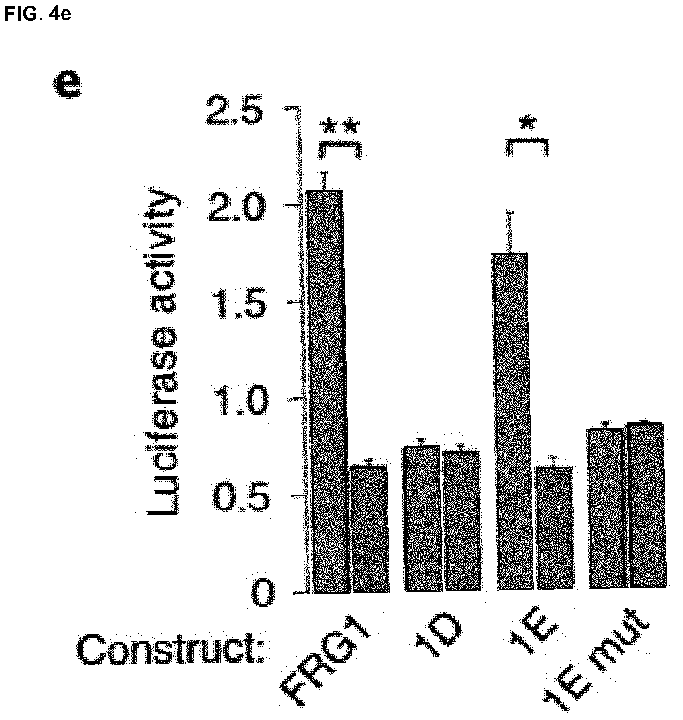

FIG. 4 shows that the stem-loop structure of HCMV RNA2.7 is the cis-acting RNA element responsible for TENT4-dependent regulation. (a) RT-qPCR of indicated RNAs after formaldehyde crosslinking and immunoprecipitation with anti-TENT4A antibody in RNA2.7-transfected HEK293T cells. Data are represented as mean±s.e.m. (n=3 inde-

pendent experiments). Immunoprecipation with NMG was used for normalization. **P<0.01, ***P<0.001; two-sided Student's t-test. (b) Schematic view of firefly luciferase reporter constructs possessing subregions and mutants of HCMV RNA2.7 in their 3' UTRs. Green, stem-loop and red asterisk, point mutations. Stem-loop structure of HCMV RNA2.7 and the mutation are shown on the left. In the 1E mut (1E mutation) construct, the red nucleotides in the loop and the C at the base of the loop are mutated as indicated. FRG, fragment. c-e, Firefly luciferase activity of reporters in parental and TENT4 KO cells. Data are represented as mean±s.e.m. Reporters with subregions of RNA2.7 (n=3 independent experiments) (c), partial deletion mutants of fragment 1 (1-513) of RNA2.7 (n=3 independent experiments) (d) and construct 1D, 1E and stem-loop mutant of 1E (n=3 independent experiments) (e). Luciferase activities of both renilla and control vector were used for normalization. *P<0.05, **P<0.01; two-sided Student's t-test.

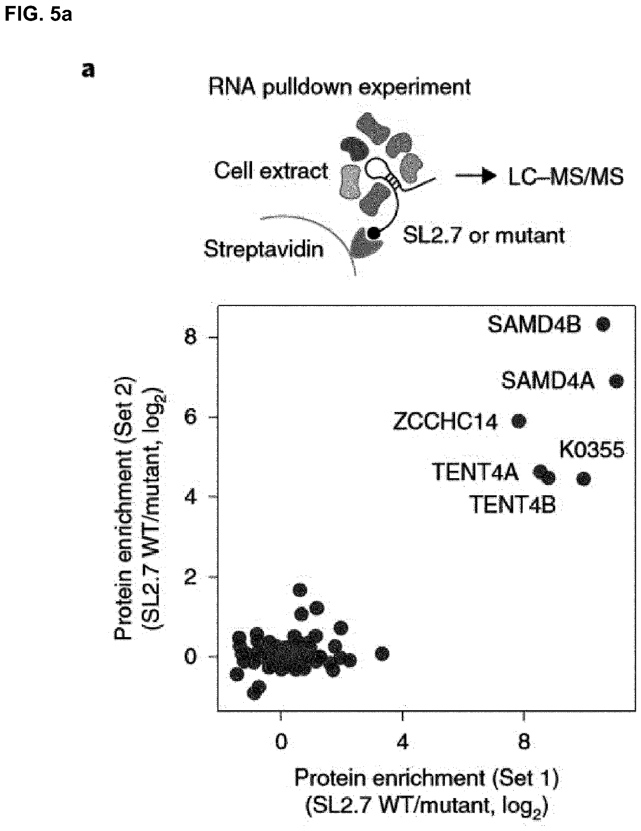

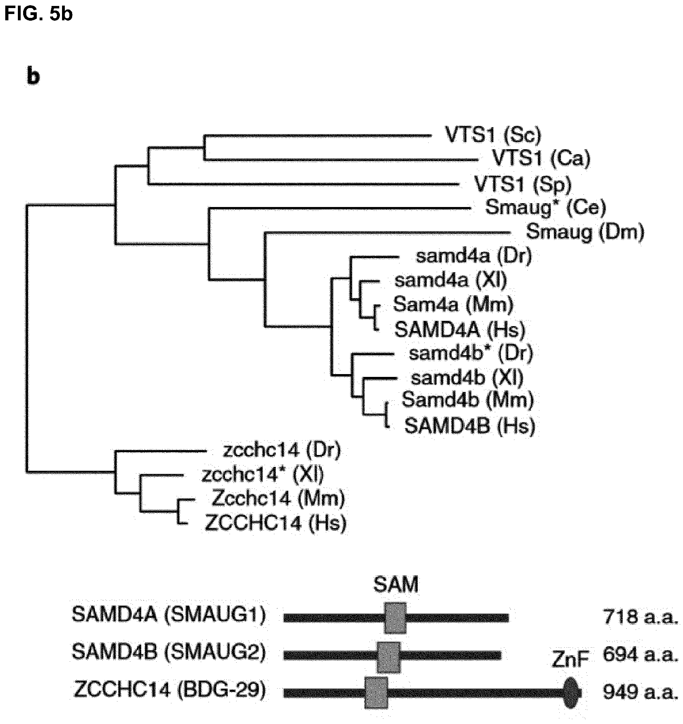

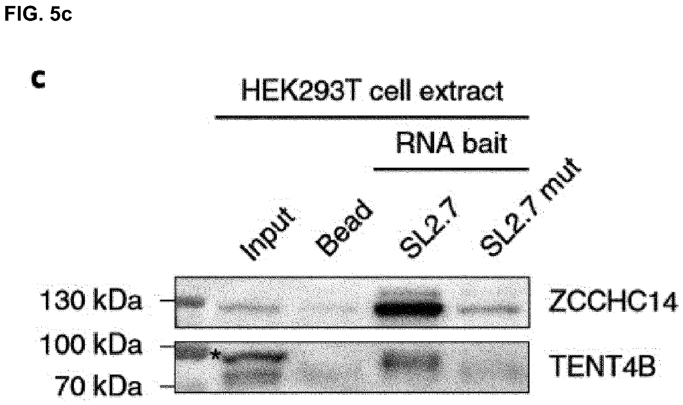

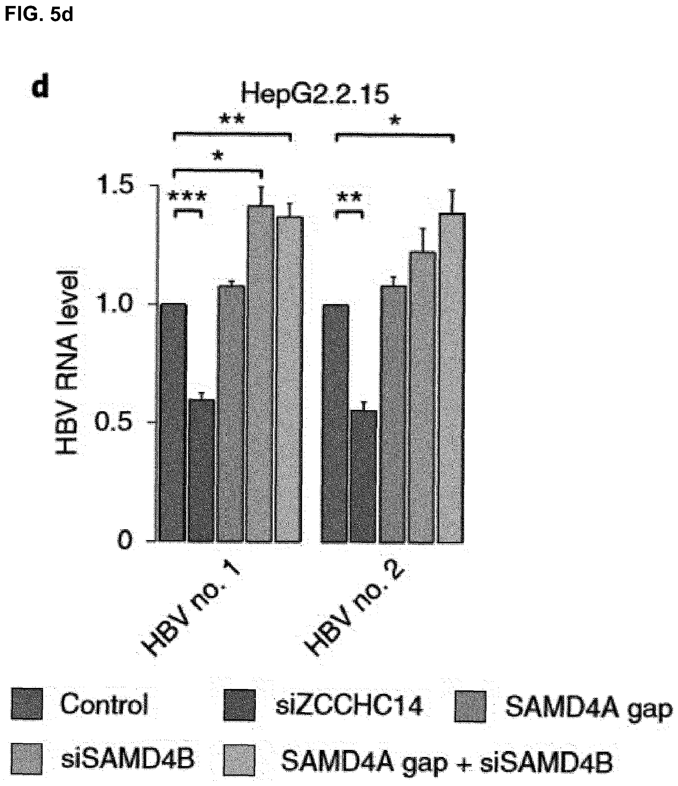

FIG. 5 shows that SAM domain-containing proteins, including ZCCHC14, bind to the stem-loop structure and regulate HBV mRNAs. a, Mass spectrometry analysis of SL2.7 RNA pulldowns from HEK293T cell lysates (n=2 mass spectrometry experiments). The SL2.7 mutant and "bead only" samples were used as negative controls. Protein enrichment score was calculated as the $\log_2$ fold change of the LFQ (label-free quantification) intensity from the SL2.7 sample over the mutant data. Spectra counts were used to filter for candidates of enrichment. b, Top, phylogenetic tree of proteins with putative RNA-binding SAM domains. Saccharomyces cerevisiae (Sc), Schizosaccharomyces pombe (Sp), Candida albicans (Ca), C. elegans (Ce), Drosophila melanogaster (Dm), Danio rerio (Dr), Xenopus laevis (Xl), Mus musculus (Mm), and Homo sapiens (Hs). The asterisks indicate manual gene name assignment based on the phylogenetic tree. UniProt IDs are listed in the method section. Bottom, domain architecture of three human proteins with SAM domains. Orange, SAM domain; purple, CCHC zinc finger (ZnF) domain. c, Pulldown of biotinylated SL2.7 from HEK293T cells, followed by western blot using the indicated antibodies. The asterisk indicates a cross-reacting band. d, HBV mRNA levels in ZCCHC14-depleted and SAMD4A/B-depleted HepG2.2.15 cells were measured by RT-qPCR using two sets of primers. Data are represented as mean±s.e.m. (n=4 independent experiments). *P<0.05, **P<0.01, ***P<0.001; two-sided t-test. Uncropped blots for c and source data for graphs in d are available online.

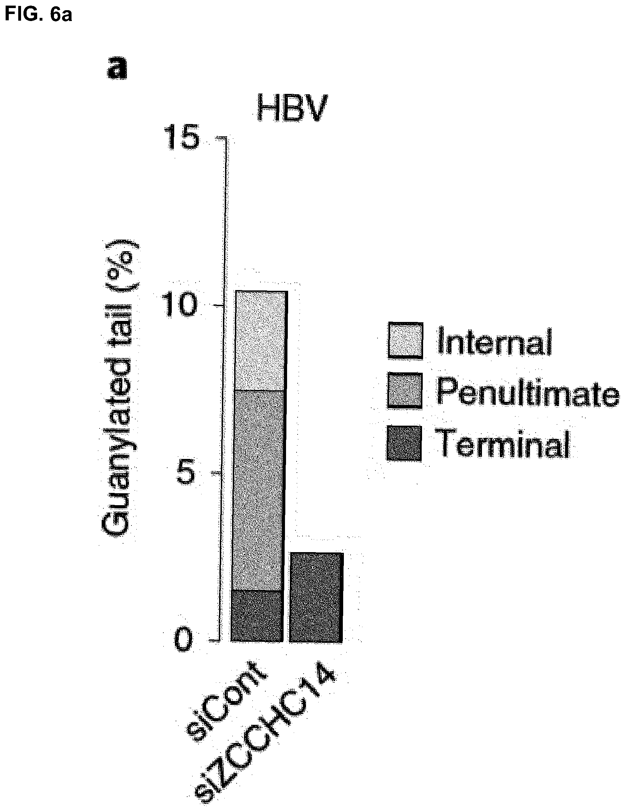

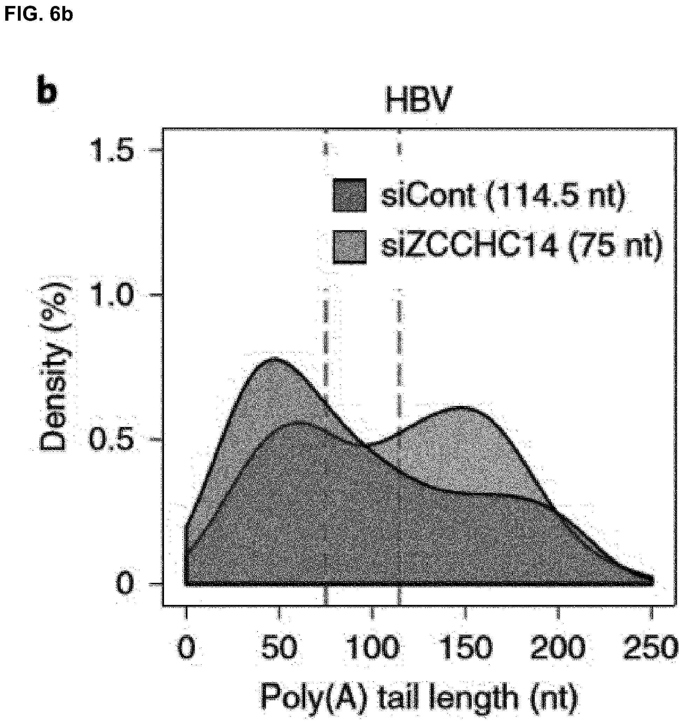

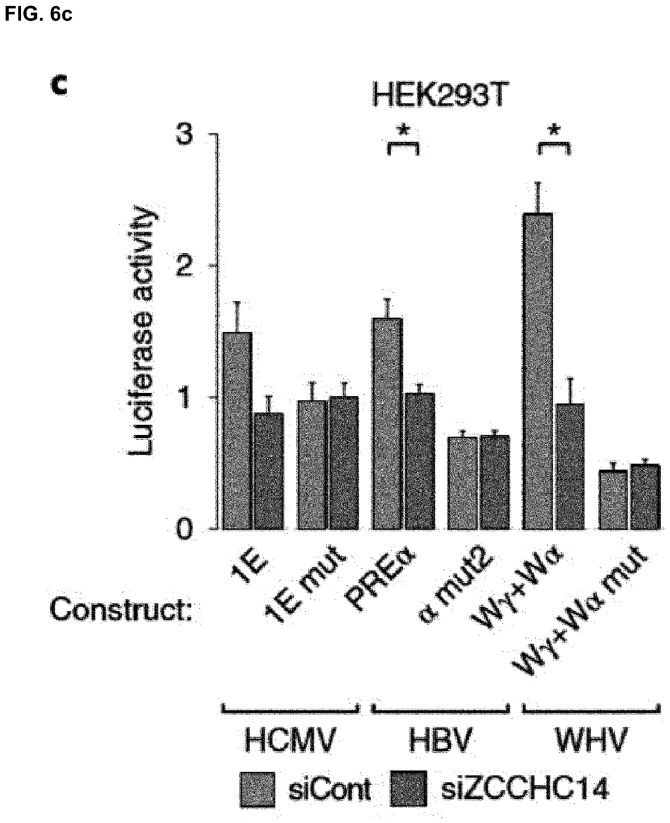

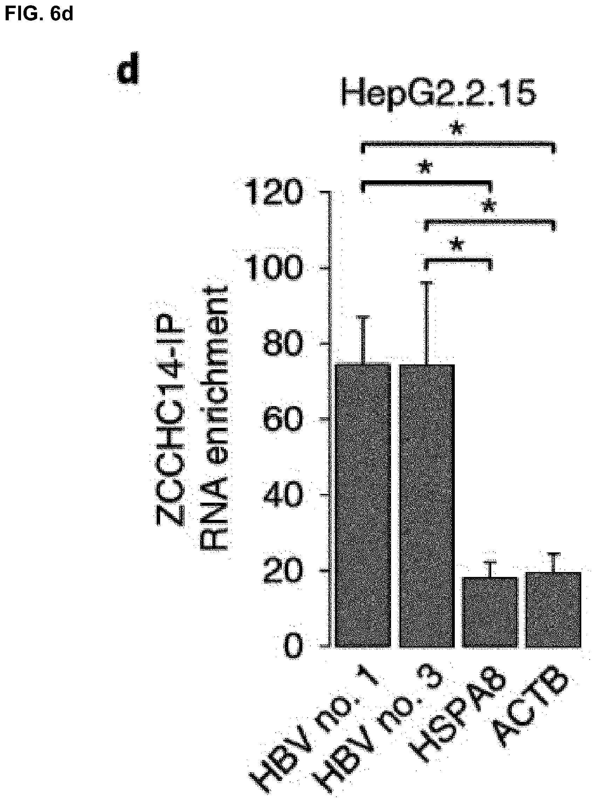

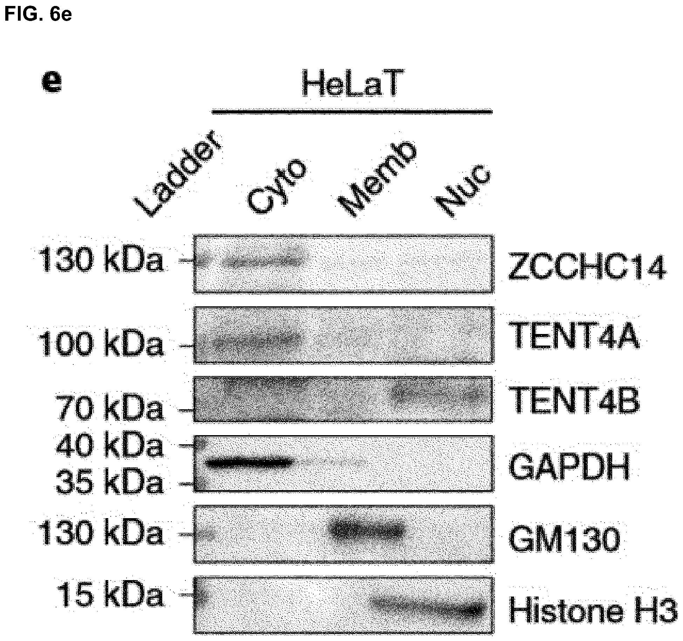

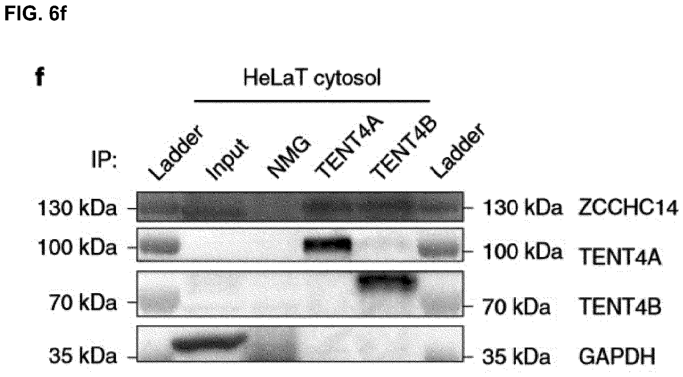

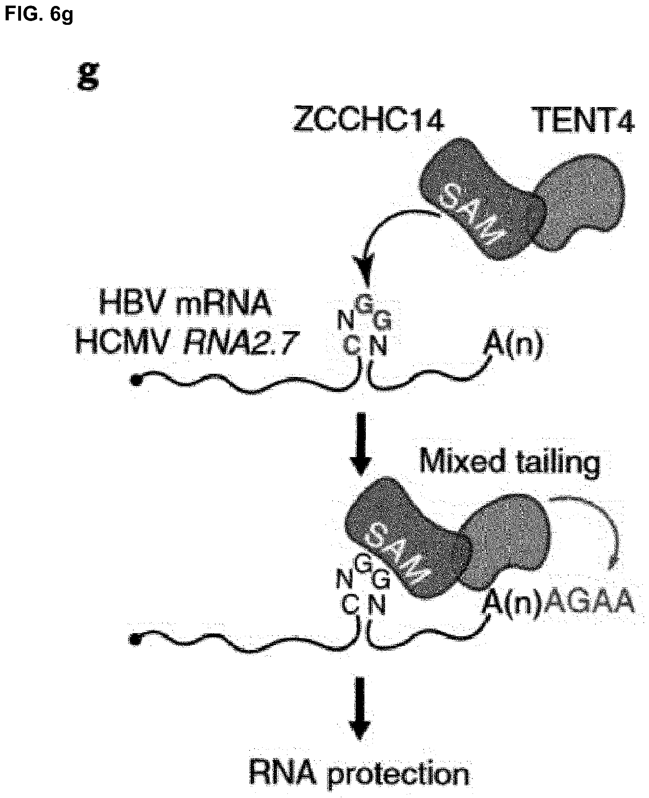

FIG. 6 shows that cytoplasmic ZCCHC14 recruits TENT4 to protect RNAs with the stem-loop structure. a, The fraction of guanylated tails of HBV mRNAs was calculated for mRNAs with a poly(A) tail length of 25 nt or more in ZCCHC14-depleted HepG2.2.15 cells. b, Global poly(A) tail length distributions of HBV mRNAs in control (gray) and ZCCHC14-depleted HepG2.2.15 cells (red). Medians are marked by dashed vertical lines and the median length is shown in parentheses. c, Firefly luciferase activity of reporters harboring HCMV RNA2.7 1E, HBV PRE$\alpha$, WHV WPRE (W$\gamma$+W$\alpha$) and their respective mutants (1E mut, $\alpha$ mut2 and W$\gamma$+W$\alpha$ mut) in ZCCHC14-depleted HEK293T cells. Data are represented as mean±s.e.m. (n=4 independent experiments for PRE$\alpha$ and $\alpha$ mut2, n=3 independent experiments for all others). Importantly, the WPRE mutant reporter (W$\gamma$+W$\alpha$ mut) consists of point mutations in both WPRE$\alpha$ stem-loop structures (Supplementary FIG. 6b). Luciferase activities of both renilla and control vector were used for normalization. *P<0.05; two-sided Student's t-test. d, RT-qPCR of indicated RNAs after immunoprecipitation with anti-ZCCHC14 antibody in HepG2.2.15 cells. Data are represented as mean±s.e.m. (n=3 independent experiments). Immunoprecipitation with normal rabbit IgG was used for normalization. *P<0.05; two-sided Student's t-test. e, Localization of ZCCHC14 protein was examined by subcellular fractionation and western blotting in HeLaT cells: cytoplasm (Cyto), membrane (Memb) and nucleus (Nuc). GAPDH (cytoplasm), GM130 (Golgi) and Histone H3 (nucleus) were used as fraction markers. f, Immunoprecipitation with anti-TENT4A and anti-TENT4B followed by western blot was used to measure the physical interaction between TENT4A/B and ZCCHC14 in HeLaT cytosol extracts. Cell extracts were treated with RNase A. GAPDH was used as a loading control. g, Proposed model. HBV mRNAs and HCMV RNA2.7 recruit the TENT4-ZCCHC14 complex via the CNGGN pentaloop to induce targeted mixed tailing, which subsequently protects the viral RNAs from cellular decay factors.

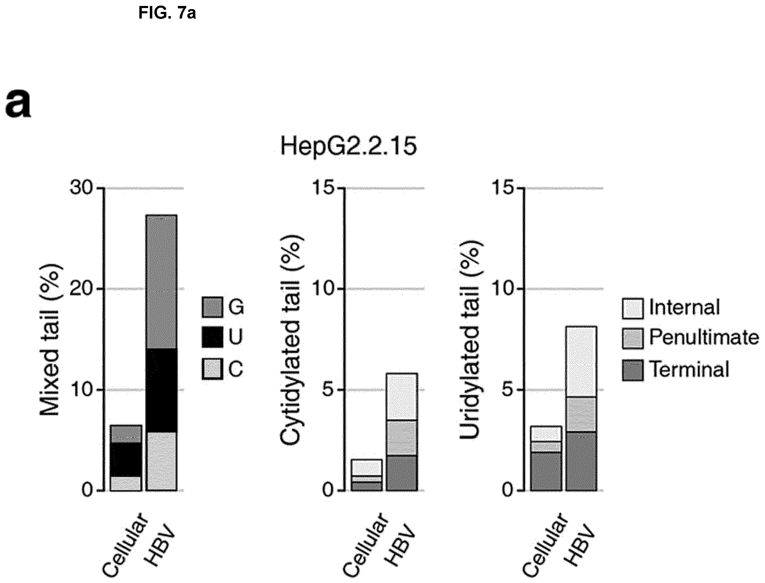

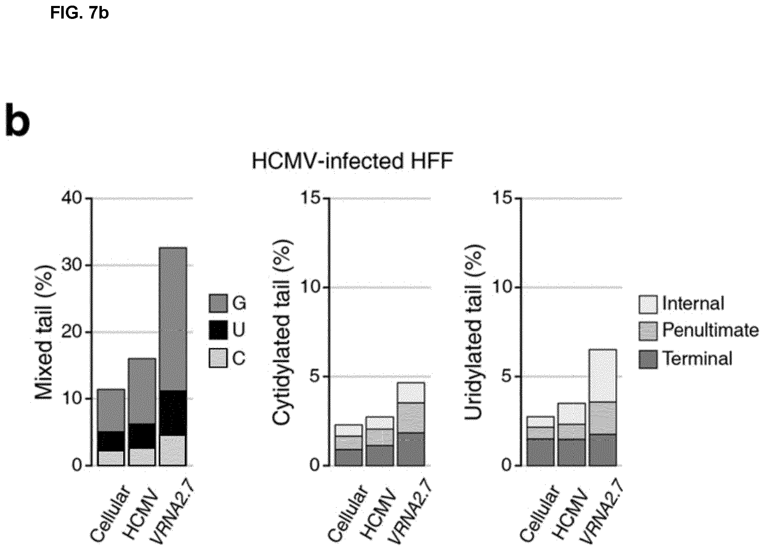

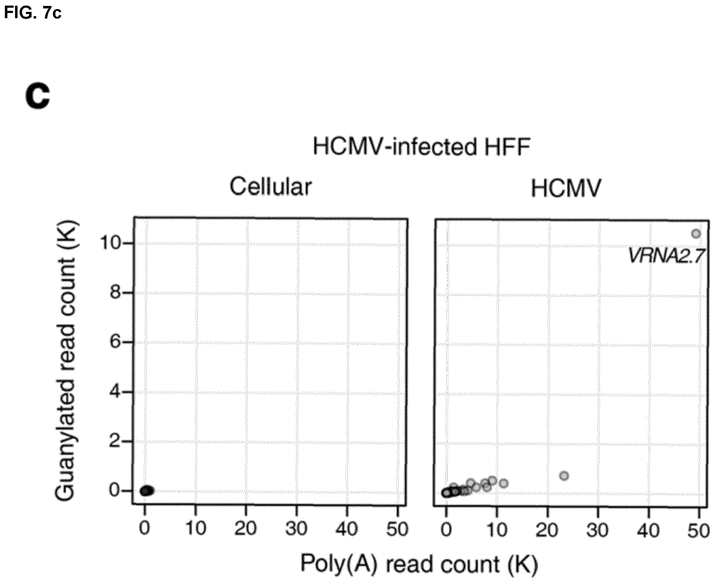

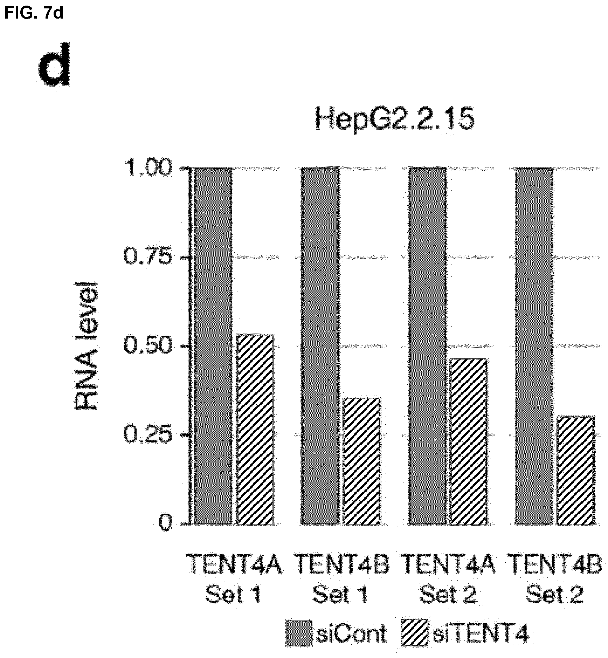

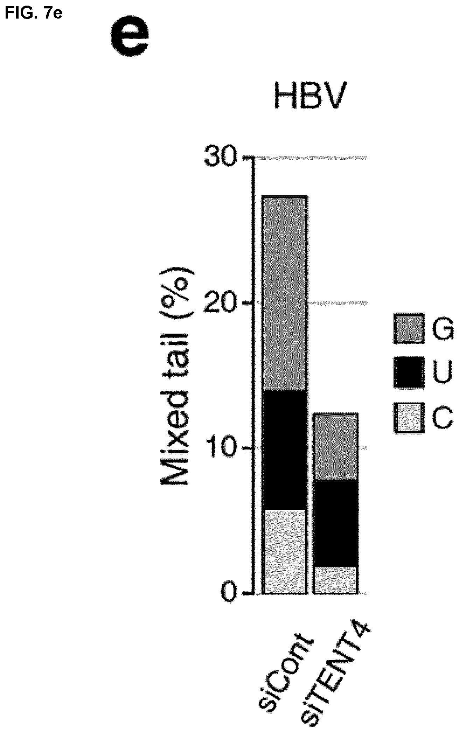

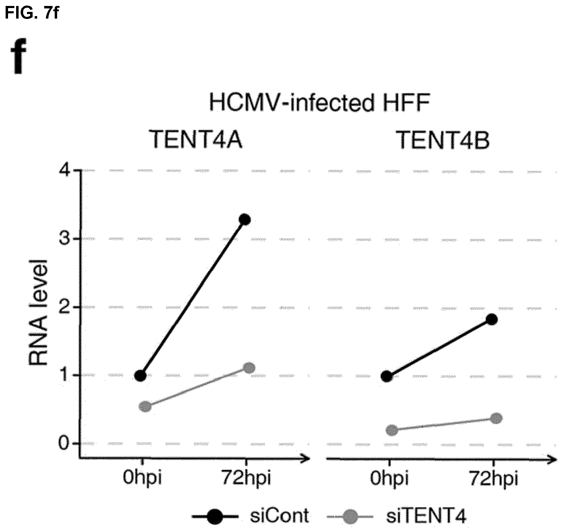

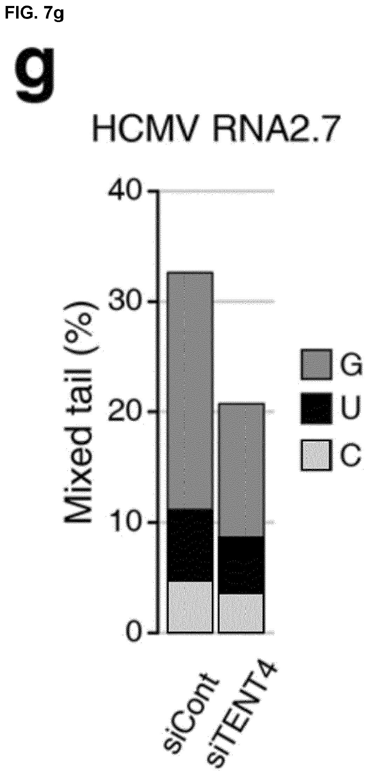

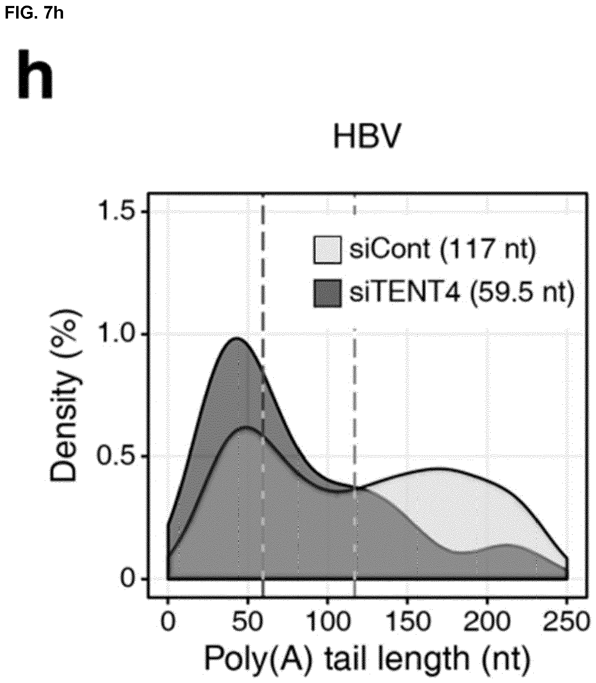

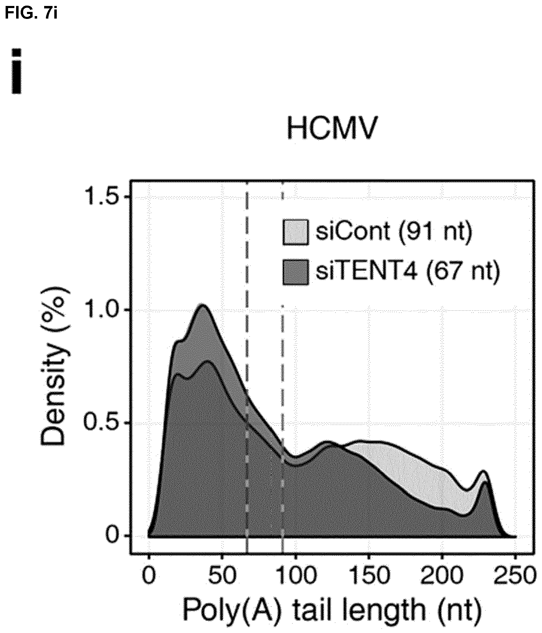

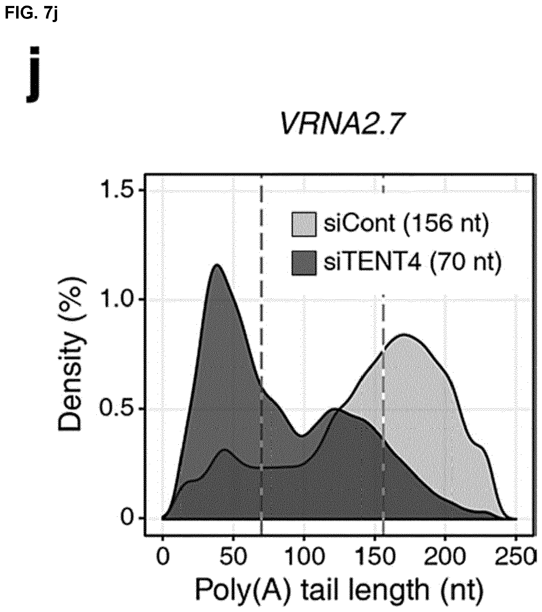

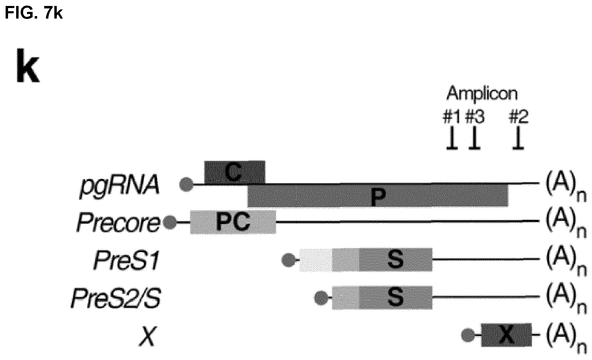

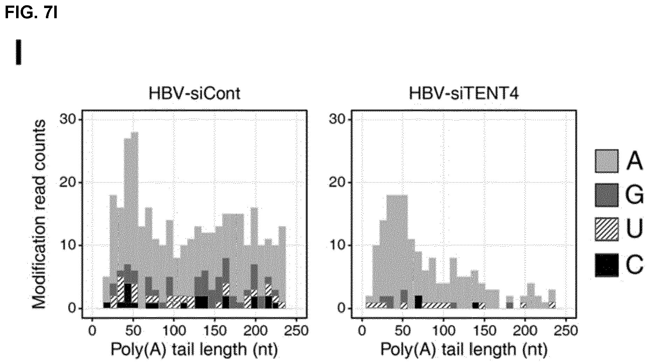

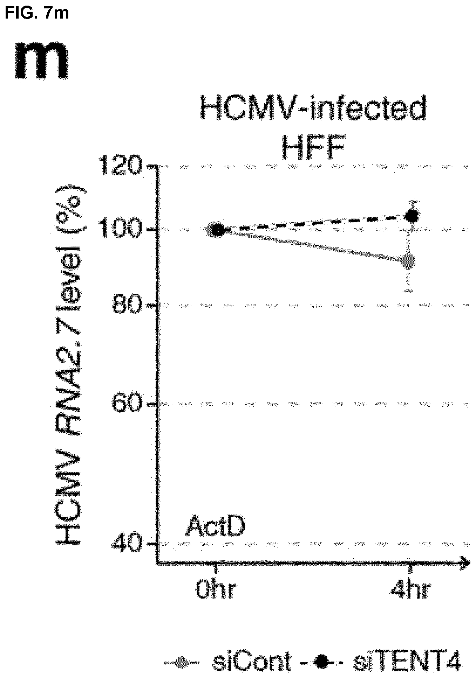

FIG. 7 shows extensive 3' end tail modification of viral RNAs. a, HBV mRNAs are highly mixed tailed (G: guanylation, U: uridylation, C: cytidylation) in HepG2.2.15 cells. Fraction of each modification includes their respective terminal and internal modifications. Fraction of cytidylated and uridylated tails of viral mRNAs were calculated for those with poly(A) tail length ≥25 nt (n=2 TAIL-seq experiments). b, HCMV RNAs are also highly mixed tailed in HCMV-infected HFF cells. HCMV RNA2.7 (VRNA2.7) is substantially mixed tailed in particular. (n=1 TAIL-seq experiment) c, Gene-level analysis of guanylated tails of cellular (grey) and viral (yellow) mRNAs in HCMV-infected HFF cells. d, siRNA knockdown confirmation by RT-qPCR. e, Decrease in the fraction of mixed tail of HBV mRNAs in TENT4-depleted HepG2.2.15 cells. f, TENT4A/B expression is induced in HCMV-infected HFF cells. TENT4A and TENT4B RNA levels in HCMV-infected HFF cells were measured by RT-qPCR (n=1). g, Decrease in the fraction of mixed tail of HCMV RNA2.7 in TENT4-depleted HCMV-infected HFF cells. h, HBV mRNAs exhibit shorter poly(A) tail length in TENT4-depleted HepG2.2.15 cells. The medians are marked by dashed vertical lines and shown in parentheses. i, Global poly(A) tail length distribution of viral mRNAs after TENT4 depletion in HCMV-infected HFF cells. j, Poly(A) tail length distribution of HCMV RNA2.7 after TENT4 depletion. k, HBV transcripts and their respective CDS regions are shown. Bars indicate the position of RT-qPCR amplicons (#1-3). 1, Tail modifications of HBV mRNAs across poly(A) tail length in TENT4-depleted HepG2.2.15 cells. m, Half-life of HCMV RNA2.7 was measured by RT-qPCR (n=3 independent experiments) in HCMV-infected HFF cells after TENT4 depletion. GAPDH mRNA was used for normalization. Mean was calculated.

Data for graphs in a-b, d-g, and m are available as source data.

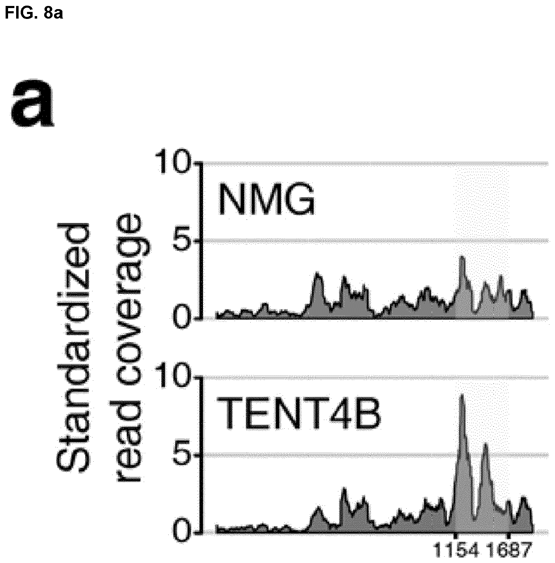

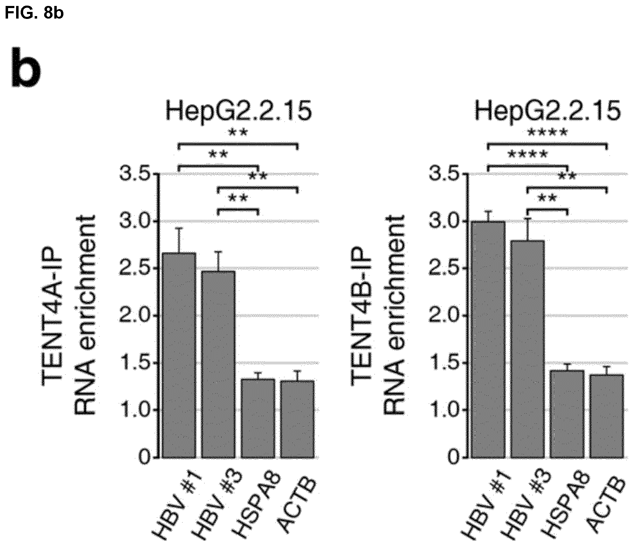

FIG. 8 shows analysis and confirmation of TENT4A/B and HBV mRNA interaction. a, TENT4B-bound RNA fragments are again enriched within the PRE region of HBV (highlighted in yellow). Standardized read coverage of fCLIP-seq libraries across the HBV genome (NCBI: U95551.1). X-axis scale is the same as in FIG. 2a. b, Immunoprecipitation with anti-TENT4A and anti-TENT4B followed by RT-qPCR was used to measure the enrichment of TENT4-bound RNA in HepG2.2.15 cells (n= 3 independent experiments). Immunoprecipitation with normal mouse IgG was used for normalization. Mean was calculated and Error bars represent SEM. **P<0.01, ****P<0.0001; two-sided t test. Data for graphs in b is available as source data.

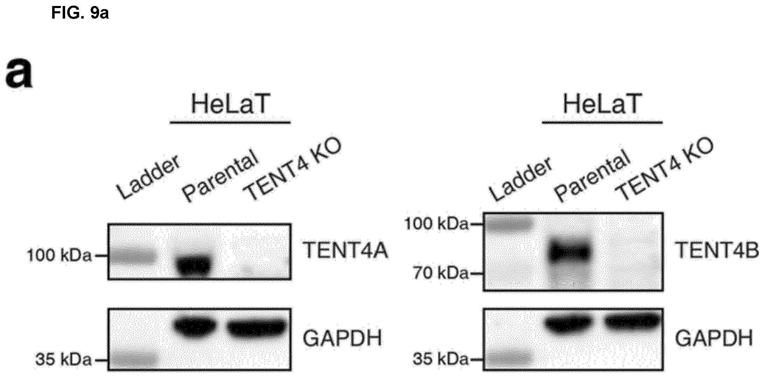

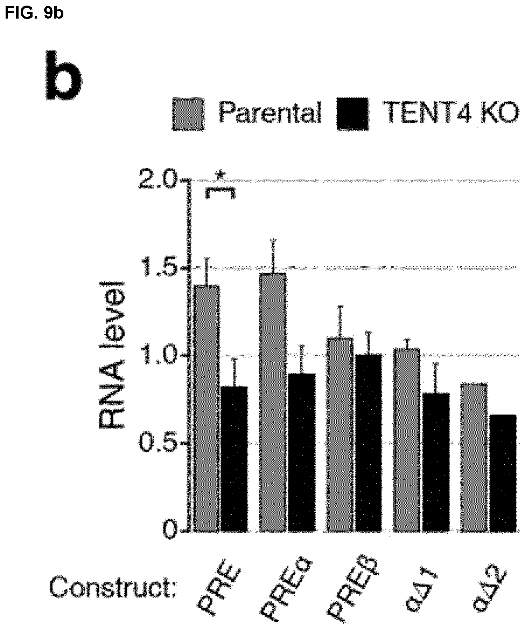

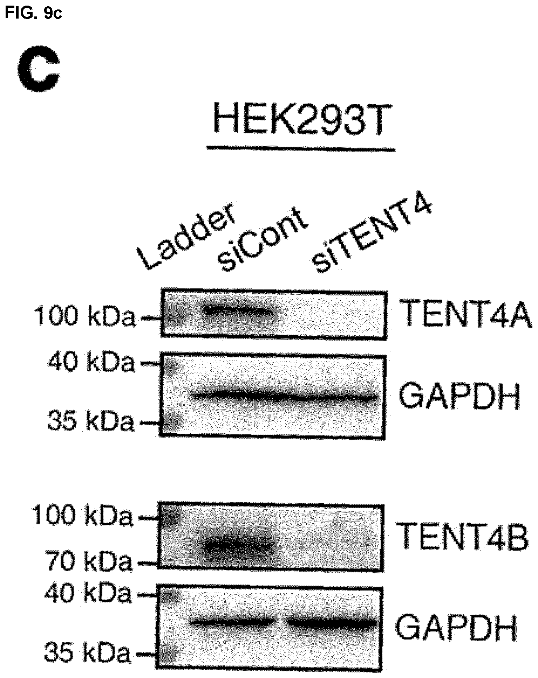

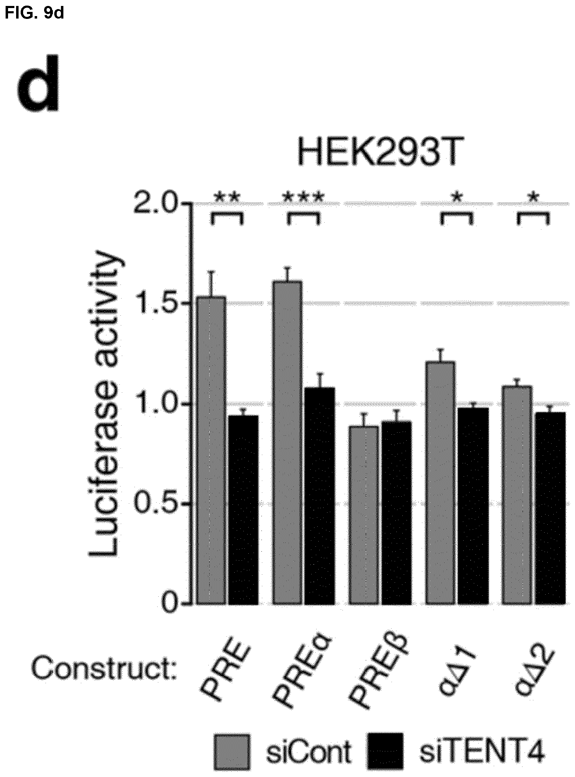

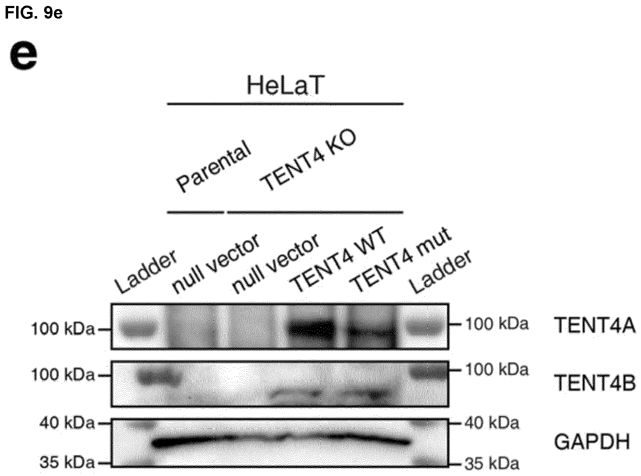

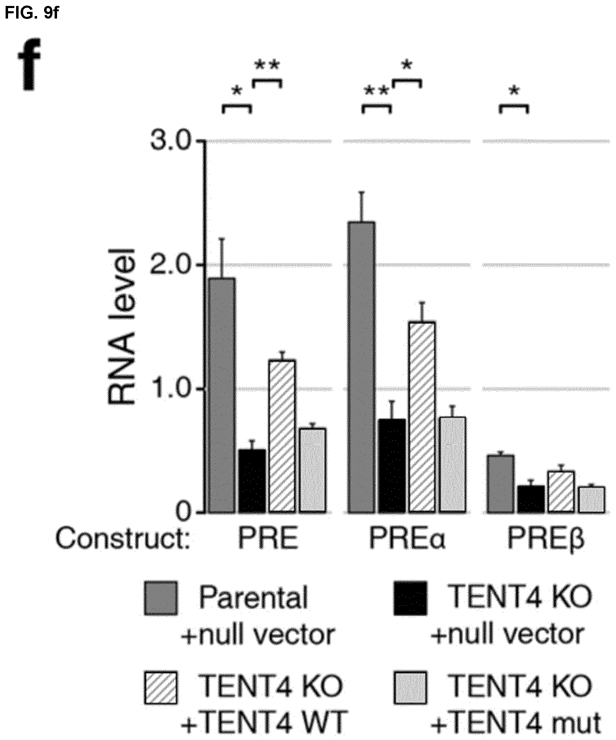

FIG. 9 shows confirmation of TENT4-dependent regulation of PRE reporters. a, Knockout confirmation by western blotting. GAPDH was used as a loading control. b, RNA levels of firefly reporters harboring subregions of PRE were measured by RT-qPCR (PRE: PREα: PREβ, n=4; αΔ1, n=2; αΔ2, n=1 independent experiment). mRNA levels of both renilla and control vector were used for normalization. Mean was calculated and Error bars represent SEM. *P<0.05; two-sided t test. c, siRNA knockdown confirmation by western blotting in HEK293T cells. GAPDH was used as a loading control. d, Firefly luciferase activity of PRE reporter constructs in TENT4-depleted HEK293T cells (PRE: PREα: PREβ, n=7; αΔ1, n=6; αΔ2, n=5 independent experiments). Luciferase activities of both renilla and control vector were used for normalization. Mean was calculated and Error bars represent SEM. *P<0.05, **P<0.01, ***P<0.001; two-sided t test. e, Over-expression confirmation by western blotting in KO cells. GAPDH was used as a loading control. f, RNA levels of firefly reporters used in rescue experiments in TENT4 KO cells with ectopically expressed TENT4 wild-type or mutant (n=3 independent experiments) were measured by RT-qPCR (n=3 independent experiments). mRNA levels of both renilla and control vector were used for normalization. Mean was calculated and Error bars represent SEM. *P<0.05, **P<0.01; two-sided t test. Uncropped blots for panels a, c, and e and data for graphs in b, d, and f are available as source data.

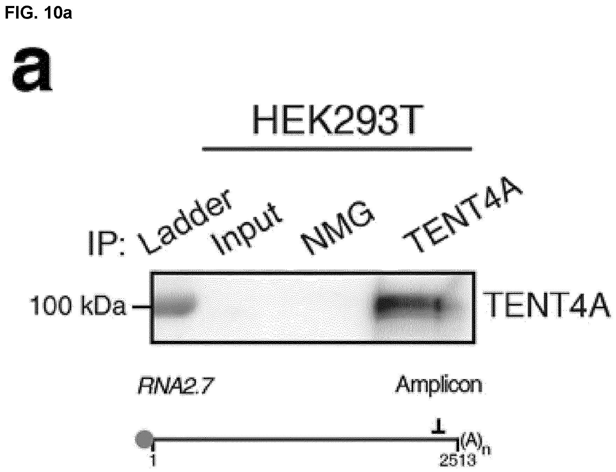

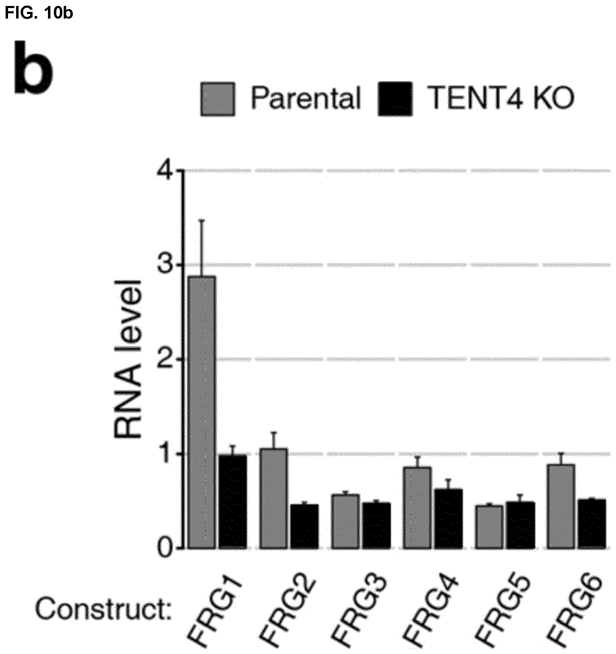

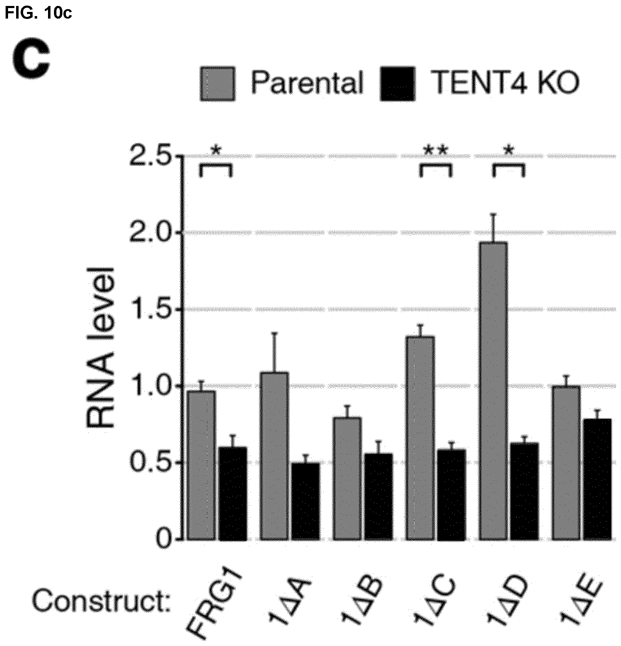

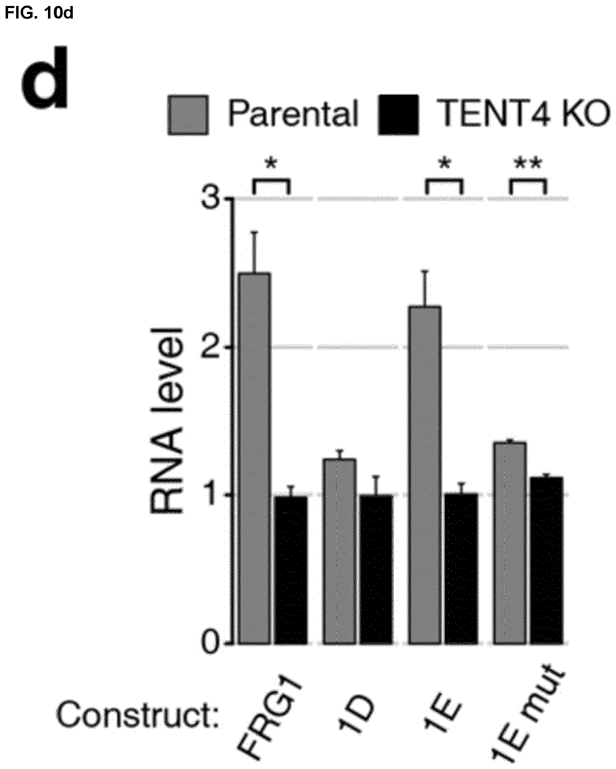

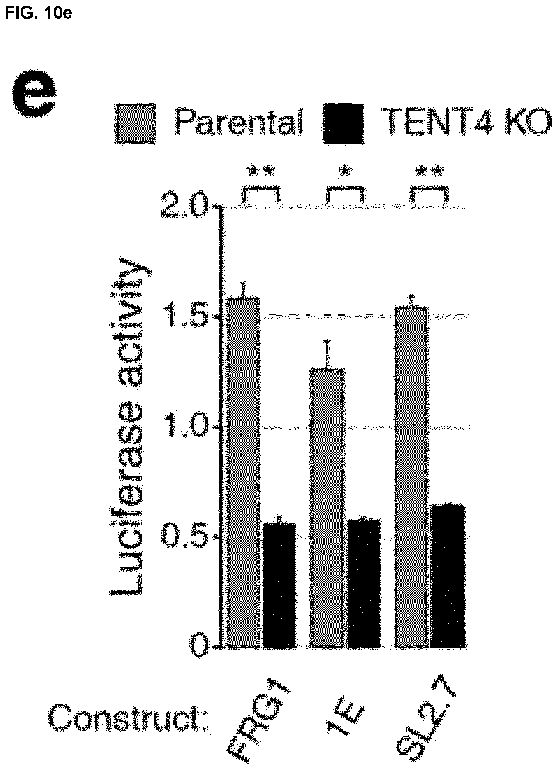

FIG. 10 shows confirmation of TENT4-dependent regulation of RNA2.7 reporters. a, Immunoprecipitation with anti-TENT4A was confirmed by western blotting in RNA2.7-transfected HEK293T cells. Normal mouse IgG (NMG) was used as a negative control. RNA2.7 transcript is shown with bar indicating the position of RT-qPCR amplicon. b-e, RNA levels of firefly reporters harboring b, subregions of RNA2.7 (n=3 independent experiments), c, deletion constructs of fragment 1 (1-513) of RNA2.7 (n=3 independent experiments), d, construct 1D (314-413), 1E (414-513) and stem-loop mutant 1E (n=3 independent experiments) and e, construct SL2.7 (414-463) and controls (n=3 independent experiments) in parental and TENT4 KO cells. mRNA levels of both renilla and control vector were used for normalization. Mean was calculated and error bars represent SEM. *P<0.05, **P<0.00.0; two-sided t test. Uncropped blots for panel a and data for graphs in b-e are available as source data.

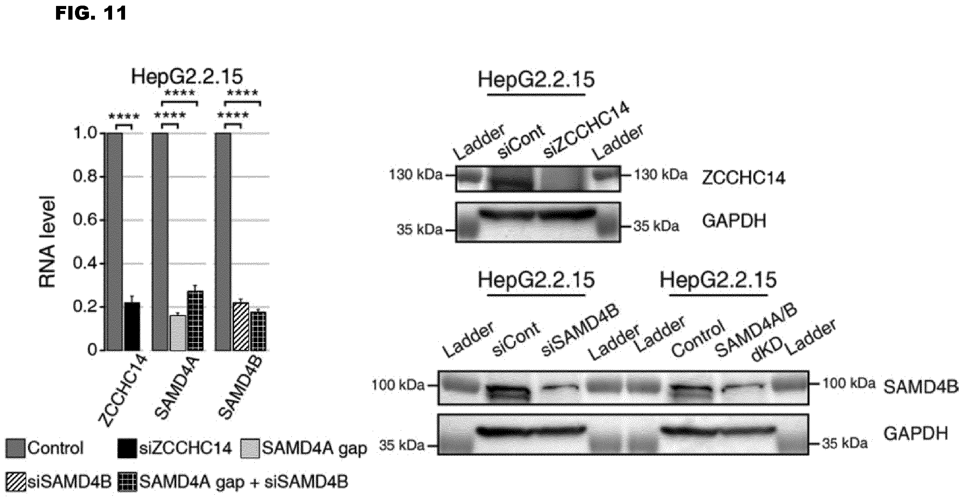

FIG. 11 shows confirmation of ZCCHC14 and SAMD4A/B knockdown. siRNA knockdown confirmation by RT-qPCR (n=4 independent experiments) and western blotting in HepG2.2.15 cells. Mean was calculated and Error bars represent SEM. ****P<0.0001; two-sided t test. GAPDH was used as a loading control.

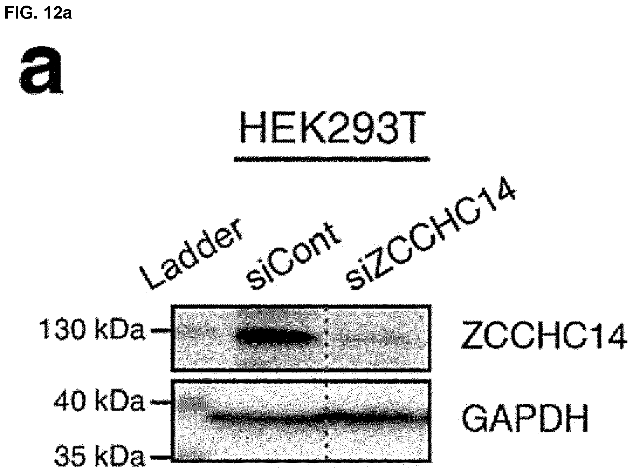

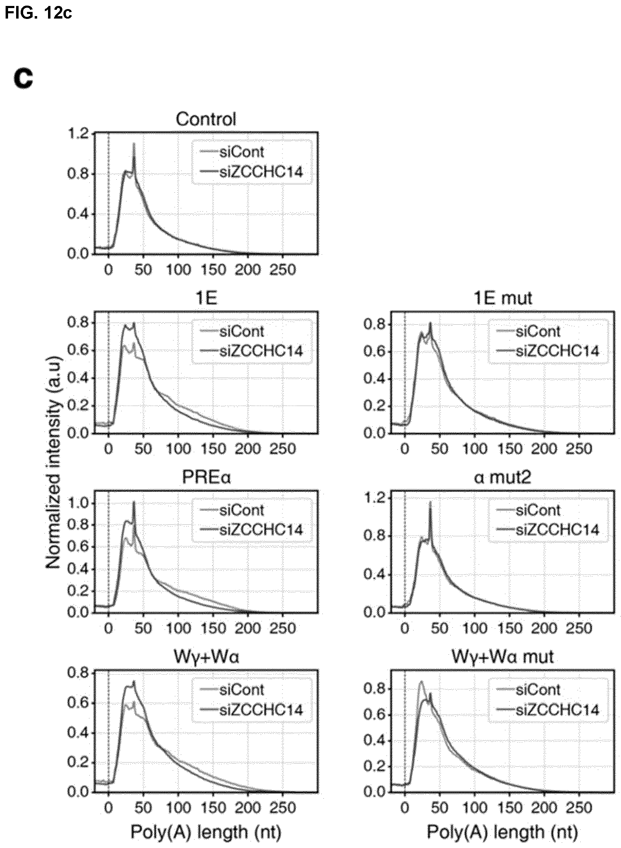

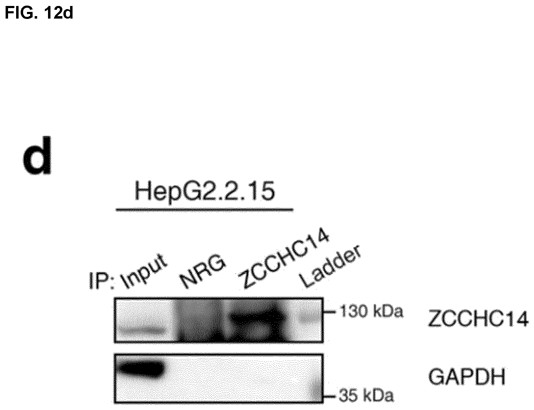

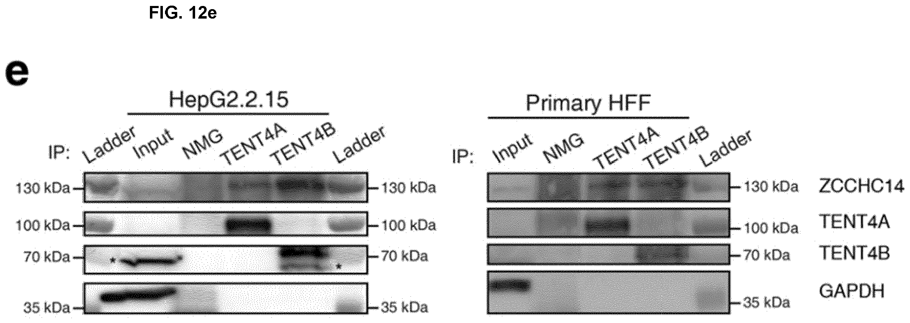

FIG. 12 shows confirmation of ZCCHC14 knockdown, stem-loop-dependent tail regulation, and immunoprecipitation. a, siRNA knockdown confirmation by western blotting in ZCCHC14-depleted HEK293T cells. GAPDH was used as a loading control. The dashed line indicates discontinuous lanes from the same gel. b, Stem-loop structures of WPREα and their respective mutants. c, Distributions of poly(A) tail length of reporter RNAs measured by Hire-PAT assay. The reporter constructs were transfected into HEK293T cells after ZCCHC14 depletion. d, Immunoprecipitation with anti-ZCCHC14 was confirmed by western blotting in HepG2.2.15 cells. Normal rabbit IgG (NRG) was used as a negative control. e, Immunoprecipitation with anti-TENT4A and anti-TENT4B followed by western blotting was used to measure the physical interaction between TENT4A/B and ZCCHC14 in HepG2.2.15 and primary HFF cells. Cell extracts were treated with RNase A. Normal mouse IgG (NMG) was used as a negative control. The asterisks indicate cross-reacting bands. Uncropped blots for panel a and d-e are available as source data.

FIG. 13 shows a stem-loop structure containing a pentaloop.

The insertion of the stem-loop structure of the present disclosure into a target RNA sequence may be performed using conventionally well-known methods without particular limitations.

Best Mode for Carrying out the Invention

A better understanding of the present disclosure may be obtained via the following examples which are set forth to illustrate, but are not to be construed as limiting the present disclosure.

EXAMPLES

Experimental Methods

EXAMPLE 1: TAIL-seq Library Preparation and Data Processing

TAIL-seq was conducted as previously described [3]. In brief, ~50 $\mu$g of DNaseI (Takara, 2270A) treated total RNA (>200 nt) was used to deplete ribosomal RNA twice by Ribo-Zero kit (Epicentre, MRZH11124 (discontinued) for primary HFF library or Illumina, TruSeq Stranded Total RNA Library Prep Human/Mouse/Rat, 20020596 for HepG2.2.15 library). The rRNA-depleted RNAs were ligated to the 3' adapter and partially fragmented by RNase T1 (Ambion). The fragmented RNAs were pulleddown with streptavidin beads (Invitrogen), 5' phosphorylated and purified by 6% urea-PAGE gel (500-1,000 nt). The purified RNAs were ligated to the 5' adapter, reverse-transcribed and amplified by PCR. The libraries were sequenced by paired-end run (51 x 251 cycles) on the Illumina platform (MiSeq) with PhiX control library v.3 (Illumina) and spike-in mixture. TAIL-seq sequencing data have been deposited to the National Center for Biotechnology Information (NCBI) Gene Expression Omnibus (GEO) database with accession number GSE146600. TAIL-seq analysis was carried out using Tailseeker v.3.1.5 [9]. Briefly, read 1 was used for gene identification, and read 2 was used to detect 3' end modifications and measure the length of the poly (A) tail. For each TAIL-seq library, read 1 was mapped to human genome GRCh38 with STAR 2.5.2b [50] and either HBV genome NCBI U95551.1 or HCMV genome NCBI GU937742.2 with bowtie2.2.6 [51]. Only TAIL-seq fragments with poly(A) tail length of at least 25 nt were used as done previously[6]. Of read 2, 3' end 10-mer sequences with terminal or internal mono-guanylation (or -cytidylation, -uridylation) were examined to estimate the fraction of nonadenosine incorporation of the poly(A) tail.

EXAMPLE 2: fCLIP-seq Library Preparation and Data Processing

fCLIP-seq was performed as previously described with minor modifications [16,52]. In brief, HepG2.2.15 cells on two 150 mm dishes were crosslinked with 0.1% paraformaldehyde (Pierce, 28906), collected and lysed. Fifteen $\mu$g of each antibody (NMG, Santa Cruz, sc-2025; TENT4A, laboratory made; TENT4B, laboratory made) was conjugated to protein A and G Sepharose beads (1:1 mix, total 20 $\mu$l, GE Healthcare, 17-5138-01 and 17-0618-01, respectively). Lysates were incubated with antibodyconjugated beads, and RNAs were purified from the eluates followed by DNaseI treatment and washing. Then, 1 $\mu$g of input RNAs or RNAs from each sample were prepared for the libraries. rRNAs were depleted twice by Ribo-Zero kit (Illumina, TruSeq Stranded Total RNA Library Prep Human/Mouse/Rat, 20020596). The rRNA-depleted RNAs were ligated to the 3' adapter and purified by 6% urea-PAGE gel (80-500 nt, corresponding to 50-470 nt RNAs that were fragmented by sonication) followed by 5' phosphorylation, 5' adapter ligation, reverse-transcription, and PCR amplification. The fCLIP-seq libraries were sequenced by paired-end run (151 x 151 cycles) on Illumina platform (MiSeq) with PhiX control library v.3 (Illumina). fCLIP-seq sequencing data have been deposited to the NCBI GEO database with accession number GSE146597.

For each fCLIP-seq library, pair-end reads were assembled with pear v.0.9.10[53], and aligned to human genome GRCh38 with STAR 2.5.2b[50] and HBV genome NCBI U95551.1 with bowtie2.2.6[51]. Raw read coverage of fCLIP-seq libraries were computed with bedtools v.2.26.0. fCLIP-seq peak clusters of cellular mRNAs were estimated using Piranha v1.2.1 [54] with a cluster size of 200 nt (-z 200), internal normalization (-n), log transformation (-l), and the NMG library as the covariate. Of note, HBV PRE spans approximately 400 nt. Enrichment scores of peak clusters were calculated by the log2 ratio of the reads of the TENT4 fCLIP-seq library and of the NMG library. The input library was used as a negative control of this analysis [55]. To account for technical background of the read coverage, the Hodges-Lehmann estimator over the HBV genome was used. Specifically, given the HBV genome of length n, the median of the following set is computed:

$$[93] \quad \{(X_i + X_j)/2 : 1 \leq i \leq j \leq n\}$$

where $X_i$ and $X_j$ are the raw read coverages at positions i and j, respectively. The raw read coverages were then normalized by this Hodges-Lehmann estimator and visualized using the ggplot2 R package.

EXAMPLE 3: Cell Culture, Transfection and Actinomycin D Treatment.

All cell lines used in this study were tested negative for mycoplasma. HeLaT was derived from HeLa (gift from C.-H. Chung at Seoul National University) with a null mutation in the TUT4 gene. HeLaT and HEK293T (gift from S. Kim at Seoul National University) cells were authenticated by ATCC (STR profiling). HepG2.2.15, HeLaT and HEK293T cells were grown in DMEM (Welgene, LM 001-05) containing 10% FBS (Welgene, S001-01). Primary HFF (ATCC SCRC-1041) cells were grown in DMEM (HyClone) containing 10% FBS (HyClone), GlutaMAX-I (Gibco), and penicillin-streptomycin (Gibco). Before transfection, primary HFF were grown in antibiotics-free media.

For combinatorial knockdown, equal amounts of small-interfering RNAs or GAPmer against target genes were mixed to have a final concentration denoted as discussed in the following. For TENT4A and TENT4B knockdown, HepG2.2.15 cells were transfected with 100 nM siRNAs by Lipofectamine RNAiMAX (Invitrogen) at days 0, 2 and 4 and collected on day 6. For ZCCHC14, SAMD4A and SAMD4B knockdown, HepG2.2.15 cells were transfected with 100 nM siRNAs or GAPmer by Lipofectamine RNAiMAX (Invitrogen) at days 0 and 2, and collected on day 4. HEK293T cells were transfected with the RNA2.7-expressing pCK vector for RNA immunoprecipitation by Lipofectamine 3000 at day 0 and collected on day 2. Primary HFF cells were transfected with 20 nM siRNAs by DharmaFECT 1 (Dharmacon) at days 0 and 2 and collected on day 5. The sequence information of siRNAs is shown in Table 1.

TABLE 1

| siRNAs (sense strand) | |
| --- | --- |
| siCont only for primary HFF | CCUACGCCACCAAUUUCGU |
| siCont | AccuTarget™ Negative Control siRNA from Bioneer Inc (Seoul, South Korea) |
| siTENT4A-1 | CUACGGUACCAAUAAUAAA |
| siTENT4A-2 | GGAAGAAUCAUCAAAGUAA |
| siTENT4A-3 | CCAAACAGAGACGCCGAAA |
| siTENT4A-4 | GCGAAUAGCCACAUGCAAU |
| siTENT4A only for HepG2.2.15 | ON-TARGETplus SMART pool (Dharmacon) |
| siTENT4B-1 | GGACGACACUUCAAUUAUU |
| siTENT4B-2 | GGCCUUUGAUUAUGCCUAC |
| siTENT4B-3 | GCGCUGACGUCCAGAUAUU |
| siTENT4B-4 | CCUAUUGCAGAGGGACCUU |
| siZCCHC14-1 | GCAUUUUAUGUGGAGCGAA |
| siZCCHC14-2 | CCUUCUCACGUGUUGAAAA |
| siZCCHC14-3 | GAAUAAAUUUGAGUCUCUU |
| siSAMD4A-1 | GGAUAUCGACAGCAAAGAA |
| siSAMD4A-2 | CAUCAUGAAACAAGGAAGA |
| siSAMD4B-1 | CACUAGAGAUGCAGAACUA |
| siSAMD4B-2 | CCUACUCAAUCGAGAGCAA |
| siSAMD4B-3 | AGGAGAACAUCACCAGUUA |

The present inventors purchased siRNAs against TENT4A from ON-TARGETplus SMARTpool (Dharmacon) and GAPmer against SAMD4A from Antisense LNA GapmeR Standard (Qiagen, 339511 LG00236046-DDA) for better knockdown efficiency in HepG2.2.15. Actinomycin D (Sigma, A9415, 4 $\mu$g ml-1) was added to HepG2.2.15 cells to block transcription and cells were gathered at the denoted time.

EXAMPLE 4: HCMV Infection

Infectious HCMV particles were generated by transfecting HCMV Toledo BAC DNAs (received from T. Shenk, Princeton University) into primary HFF cells by electroporation (Invitrogen, Neon). When a 100% cytopathic effect was observed, cell culture supernatants were gathered, centrifuged to remove cell debris, and stored at -80°C in 1 ml aliquots. To titrate the viral stocks, primary HFFs grown on cover glass were inoculated with diluted virus stocks for 1 hour and fixed with 3.7% formaldehyde at 24 hours post inoculation. Cells were permeabilized using 0.1% Triton X-100, incubated with blocking buffer (2% bovine serum albumin in phosphate-buffered saline), stained with HCMV IE1 antibody (MAB810R; Millipore) followed by FITC-conjugated anti-mouse antibody (115-095-146; Jackson Laboratories), and mounted with DAPI-containing solution (H-1200; Vector Laboratory). The number of HCMV IE1-positive cells were counted and the multiplicity of infection (MOI) was determined by calculating the ratio of IE1-positive cells to total cells. For HCMV infection, primary HFF cells were incubated with HCMV (MOI, 2) diluted in serum-free DMEM for 1 hour, washed with PBS, and incubated in DMEM for further culture.

EXAMPLE 5: Plasmid Construction

Previously described wild-type TENT4A and TENT4B isoforms (792 and 666 aa, respectively) [6] except the FLAG tag were used. A point mutation for catalytic dead mutant was introduced (D352A for TENT4A, D326A for TENT4B, respectively). For PRE reporter constructs, PRE (1,154-1,687 bp), PREα (1,154-1,351 bp), PREβ (1,352-1,687 bp), αΔ1 (1,276-1,351 bp), and αΔ2 (1,154-1,275 bp) were amplified from HepG2.2.15 complementary DNA and introduced into the 3' UTR region of firefly luciferase mRNA sequence in pmirGLO-3XmiR-1 vector[6]. Point mutations for the La-binding motif and stem-loop α were the same as the previously described mutants[30]. α mut1, α mut2, and α mut3 were generated by PCR-directed mutagenesis. For WPRE reporter constructs, WPRE (1,095-1,670 bp), Wγ+Wα (1,095-1,507 bp), Wα+Wβ (1,300-1,670 bp), Wγ (1,093-1,299 bp), Wα (1,300-1,507 bp), and Wβ (1,508-1,670 bp) were amplified from pLenti-CMV vector (Addgene, 17492) and introduced into 3' UTR of firefly luciferase in pmirGLO-3Xmir-1 vector. Point mutations for the CNGGN pentaloops of WPREα were designed in a similar manner to the PRE mutants. For RNA2.7 construct, FRG1 (1-513 bp), FRG2 (414-913 bp), FRG3 (814-1,313 bp), FRG4 (1,214-1,713 bp), FRG5 (1,614-2,113 bp), FRG6 (2,014-2,513 bp), 1ΔA (1-513 bp, Δ1-100 bp), 1ΔB (1-513 bp, Δ101-200 bp), 1ΔC (1-513 bp, Δ201-300 bp), 1ΔD (1-513 bp, Δ301-400 bp), 1ΔE (1-513 bp, Δ401-513 bp), 1D (301-400 bp), 1E (401-513 bp), and SL2.7 (414-463 bp) were amplified from HCMV-infected primary HFF cDNA and introduced into the 3' UTR region in pmirGLO-3Xmir-1 vector. The 1E and SL2.7 mutant constructs were generated in the same way as the PRE mutants. For RNA immunoprecipitation, RNA2.7 (1-2,513 bp) were amplified from pmirGLO-3Xmir-1 vector and subcloned into a pCK vector.

EXAMPLE 6: TENT4A and TENT4B Knockout Cell Preparation

For ablation of TENT4A and TENT4B, CRISPR-Cas genome editing was carried out as previously described[56] with minor adaptations that T7 Endonuclease I (NEB, M0302) was used instead of Surveyor nuclease. The 6E4 HeLaT cells on 24-well plate were transfected by using Metafectene (Biontex, T020) with 300 ng pSpCas9(BB)-2A-GFP-px458 plasmid (Addgene no. 48138) with single-guide RNA (agccacgttgtgcttccgcg, PAM sequence is GGG) against TENT4A first. This HeLaT parental cell was derived from HeLa and contained a null mutation in the TUT4 gene. After single cell screening and Sanger sequencing to confirm knockout, parental and changed genomic sequences are listed in Table 2, and inserted sequences are marked in bold and underlined.

TABLE 2

| Genomic sequences of TENT4A and TENT4B KO HeLaT cells | |
| --- | --- |
| Parental | GAGCAAGCCCTGCGGAAGCACAACGTGGCT |
| TENT4A KO | GAGCAAGCCCTGCGGGAAGCACAACGTGGCT |
| | GAGCAAGCCCTGCGCAAGATGGACGGCACCGAGGAACTGCTCGTGAAGCTGAACAGAGAGGACCTGCTGCGGAAGCAGCGGACCTTCGACAACGGCAGCATCCCCCACCAGATCCACCTGGGAGAGCTGCACGCCATTCTGCGGCGGCAGGAAGAAGCACAACGTGGCT |
| | GAGCAAGCCCTGCGCCGGTTCTTCCGTCTGGTGTATCTTCTTCTGGCGGTTCTCTTCAGCCGGGTGGCCTCGGCTGTTTCGCCGCTGTCGAACAGCAGGGCTCCGATCAGGTTCTTCTTGATGCTGTGCCGGTCGGTGTTGCCCAGCACCTTGAATTTCTTGCTGGGCACCTTGTACTCGTCGGAAGCACAACGTGGCT |
| Parental | TAGTGACATCGACCTAGTGGTGTTTGGGAA |
| TENT4B KO | TAGTGACATCGACCTAGT--TGTTTGGGAA |
| | TAGTGACATCGACCTAGT-A-AGTTTGGGAA |

(continued)

| Genomic sequences of TENT4A and TENT4B KO HeLaT cells | |
|---|---|
| Parental | GAGCAAGCCCTGCGGAAGCACAACGTGGCT |
| | TAGTGACATCGACCTAGTGGTTGTTTGGGAA |

Using TENT4A knockout cells, TENT4B was further ablated with sgRNA (gacatcgacctagtggtgtt, PAM sequence is TGG) against TENT4B. Changed genomic sequences are also listed in Table 2, and inserted and deleted sequences are marked in red and dashes, respectively.

EXAMPLE 7: TENT4A and TENT4B Antibody Preparation

Mice were immunized with TENT4A antigen (126-571 aa from 792 aa isoform, NCBI NP_008930.2) or TENT4B antigen (106-533 aa from 666 aa isoform, NCBI XP_006721308.1) and killed. Collected splenocytes were fused with Sp2/O myeloma, and hybridoma cells producing high affinity antibodies were selected. Ascites was obtained after intraperitoneal injection of hybridoma cells to mice (Youngin Frontier Inc.), and used for the immunoprecipitation experiment.

EXAMPLE 8: Western Blotting

HepG2.2.15 cells were lysed in RIPA buffer (20 mM Tris-HCl (pH 7.5), 150 mM NaCl, 1 mM EDTA, 10% NP-40 (Sigma, 74385), 1% sodium deoxycholate (Sigma, D6750), 1% SDS (Ambion, AM9823)). Other cells were lysed in Buffer D (200 mM KCl, 10 mM Tris-HCl (pH 8.0), 0.2 mM EDTA, 0.1% Triton X-100 (Promega, H5142)). Roughly 60 $\mu$g of lysate was loaded on 10% or 8-16% (Novex) SDS-PAGE gel with the ladder (Thermo, 26616 and 26619). After transferring to a methanol-activated polyvinylidene difluoride membrane (Millipore), the membrane was blocked in PBS-T containing 5% skim milk, probed with primary antibodies and washed three times with PBS-T. Anti-mouse or anti-rabbit HRP-conjugated secondary antibodies (Jackson ImmunoResearch Laboratories) were incubated and washed three times with PBS-T. Chemiluminescence was conducted with West Pico or Femto Luminol reagents (Thermo), and the signals were detected by ChemiDoc XRS+ System (BioRad). Anti-TENT4A (1:500, Invitrogen, PA5-61302;1:250-500, Atlas Antibodies, HPA045487), anti-TENT4B (1:500, Invitrogen, PA5-60177;1:500, Atlas Antibodies, HPA042968;1:500, laboratory made), anti-ZCCHC14 (1:1,000-2,000, Bethyl Laboratories, A303-096A), anti-GAPDH (1:1,000-2,000, Santa Cruz, sc-32233), anti-SAMD4B (1:500, Invitrogen, PA5-53490), anti-GM130 (1:500, BD Bioscience, 610822) and anti-Histone H3 (1:2,000, Cell signaling, 4499) were used as the primary antibodies.

EXAMPLE 9: RT-qPCR

Total RNA was extracted using Trizol reagent (Invitrogen, 15596018) or Maxwell 16 LEV simplyRNA Tissue kit (Promega, AS1280), treated with DNaseI, purified with RNeasy MinElute Cleanup Kit (Qiagen), and reverse-transcribed with RevertAid reverse transcriptase (Thermo) and oligo dT for luciferase assay samples or random hexamer (Invitrogen) for other samples. mRNA levels were measured with SYBR Green assays (Thermo) and StepOnePlus Real-Time PCR System (Applied Biosystems) or QuantStudio 3 (Applied Biosystems). The list of RT-qPCR primers is given in Table 3.

TABLE 3

| qPCR primers | |
|---|---|
| qPCR-ZCCHC14-F | ACCCCGTCTTTAAGCAGCTC |
| qPCR-ZCCHC14-R | GGCACCGTCTCTCTGACTTC |
| qPCR-TENT4A-F | CCCACCACTTCCAGAACACT |
| qPCR-TENT4A-R | GCTTTCAAAGACGCAGTTCC |
| qPCR-TENT4B-F | TCGCAGATGAGGATTCG |
| qPCR-TENT4B-R | CTGCTCTCACGCCATTCT |
| qPCR-GAPDH-F | CTCTCTGCTCCTCCTGTTCGAC |

(continued)

| qPCR primers | |
|---|---|
| qPCR-GAPDH-R | TGAGCGATGTGGCTCGGCT |
| qPCR-FireflyLuc-F | CCCATCTTCGGCAACCAGAT |
| qPCR-FireflyLuc-R | GTACATGAGCACGACCCGAA |
| qPCR-RenillaLuc-F | CTGGACGAAGAGCATCAGG |
| qPCR-RenillaLuc-R | TGATATTCGGCAAGCAGGCA |
| qPCR-SAMD4A-F | CTGAGCAGCTGCGATGGG |
| qPCR-SAMD4A-R | GGTCTGGAGACCAAGAGCTG |
| qPCR-SAMD4B-F | TGCAATGACATCCACCTGCT |
| qPCR-SAMD4B-R | GTACTCCGACTTGGCCTCTG |
| qPCR-HBV#1-F | AACGGCCAGGTCTGTGCCAA |
| qPCR-HBV#1-R | CCGCAGTATGGATCGGCAGAG |
| qPCR-HBV#2-F | TACATAAGAGGACTCTTGG |
| qPCR-HBV#2-R | GCAGACCAATTTATGCCTAC |
| qPCR-HBV#3-F | TACTGCGGAACTCCTAGCCG |
| qPCR-HBV#3-R | TTGCGGGAGAGGACAACAGAG |
| qPCR-RNA2.7-F | CAGCTTTTCTCCCAAACCTCG |
| qPCR-RNA2.7-R | AGGAATAATCCGTGCGACCG |
| qPCR-HSPA8-F | ACCTACTCTTGTGTGGGTGTT |
| qPCR-HSPA8-R | GACATAGCTTGGAGTGGTTCG |
| qPCR-ACTB-F | CCTGTACGCCAACACAGTGC |
| qPCR-ACTB-R | ATACTCCTGCTTGCTGATCC |
| qPCR-FireflyLuc-F used for RIP-qPCR spike-in | AAGGTTGTGGATCTGGATAC |
| qPCR-FireflyLuc-R used for RIP-qPCR spike-in | GATTGTTTACATAACCGGAC |

EXAMPLE 10: RNA Immunoprecipitation

For TENT4A and TENT4B immunoprecipitation with HepG2.2.15 cells, cells from one 150 mm dish were gathered, lysed with lysis buffer (20 mM HEPES (pH 7.0-7.6) (Sigma, H0887), 4% NP-40, 100 mM KCl, 0.1 mM EDTA, 10% glycerol, 1 mM DTT, 20 U ml-1 RNase inhibitor (Ambion, AM2696), 1x protease inhibitor (Calbiochem, 535140)) on ice and then centrifuged. 0.375 $\mu$g of each antibody (NMG, Santa Cruz, sc-2025; anti-TENT4A, laboratory made; anti-TENT4B, laboratory made) was conjugated to protein A and G sepharose beads (1:1 mix, total 10 $\mu$l). Lysates were incubated with antibody-conjugated beads and then washed with wash buffer (the same lysis buffer but with 2% NP-40). After adding 10 ng of firefly luciferase mRNA to the sample as a spike-in used for normalization, RNAs were purified by TRIzol reagent, treated with DNaseI and used for RT-qPCR. A similar method was used for ZCCHC14 immunoprecipitation with HepG2.2.15 cells but also with Turbo DNaseI (Ambion, AM2239) treatment during lysis and each 10 $\mu$g of antibody (Normal rabbit IgG (Cell signaling, 2729S) and anti-ZCCHC14 (Bethyl Laboratories, A303-096A)). For TENT4A immunoprecipitation with HEK293T cells (one 100 mm dish), a similar method was used with each 6 $\mu$g of antibody (NMG and anti-TENT4A) and the cells were also crosslinked (0.1% paraformaldehyde) before collection.

EXAMPLE 11: Coimmunoprecipitation

Similar steps were used as in RNA immunoprecipitation but with the addition of Turbo DNaseI and RNase A (Thermo, EN0531). Briefly, HepG2.2.15 cells from five 150 mm dishes were lysed and used for immunoprecipitation with 17.5 $\mu$g of each antibody (NMG, anti-TENT4A, anti-TENT4B), which was conjugated to protein A and G sepharose beads (1:1 mix, total 25 μl). Primary HFF cells from two 150 mm dishes were lysed and used for immunoprecipitation with each 6 μg of antibody (NMG, anti-TENT4A, anti-TENT4B), which was conjugated to protein A and G sepharose beads (1:1 mix, total 20 μl). RNase A was added to lysates to have 0.2-0.3 μg/μl final concentration. For the coimmunoprecipitation experiment, cytosol extracts from 1 and 1/3 150 mm dishes of HeLaT cells were obtained by subcellular fractionation as described in the following, and then used for immunoprecipitation with 18 μg of each antibody (NMG, anti-TENT4A, anti-TENT4B).

EXAMPLE 12: Luciferase Assay and Transfection

The 1E5 HeLaT cells on 24-well plate were transfected with 200 ng pmirGLO plasmids for luciferase reporter assay by Lipofectamine 3000 at day 0 and collected on day 2. For the rescue experiment, 1.5E5 HeLaT parental and TENT4 KO cells on a 24-well plate were transfected with 50 ng of pmirGLO plasmids along with 20 ng of null vector or TENT4A/B (1:1 mix) plasmids by Lipofectamine 3000 at day 0 and collected on day 2. HEK293T cells were transfected with 100 nM siRNAs at Day 0 and 1.5E5 cells on 24-well plate were further transfected with 100 nM siRNAs at day 2 along with 100 or 200 ng of pmirGLO plasmids by Lipofectamine 3000. HEK293T cells were collected on day 4. For luciferase assay, cells were lysed and analyzed with the dual-luciferase reporter assay system (Promega) according to the manufacturer's instructions.

EXAMPLE 13: Subcellular Fractionation

The 2E6 HeLaT cells were collected and washed with cold PBS. To obtain cytosolic fraction, cells were lysed with 200 μl of cytosol lysis buffer (0.2 μg/μl digitonin (Sigma, D141), 150 mM NaCl, 50 mM HEPES (pH 7.0-7.6), 0.1 mM EDTA, 1 mM DTT, 20 U/ml RNase inhibitor, 1x protease inhibitor, 1x phosphatase inhibitor). After centrifugation at 2,000 relative centrifugal force at 4°C for 5 min, supernatant was collected and used as a cytosol fraction. For the membrane and nucleus fractions, subcellular protein fractionation kit (Thermo Scientific, 78840) was used according to the manufacturer's instructions.

EXAMPLE 14: Hire-PAT Assay

Hire-PAT assay and signal processing of capillary electrophoresis data were conducted as previously described[3,57] with minor adaptations that total RNAs were G/I tailed by yeast poly(A) polymerase (Thermo Scientific, 74225Z25KU). Poly(A) site of firefly luciferase gene was confirmed by Sanger sequencing, and the forward PCR primer is listed in Table 4.

TABLE 4

| Hire-PAT PCR primer | |
| --- | --- |
| Hire-PAT-FireflyLuc-F | GGACAAACCACAACTAGAATG |

EXAMPLE 15: Phylogenetic Analysis

To identify putative stem-loop binding proteins, homologs of the RNA-binding SAM domain of Drosophila Smaug (UniProtKB Q23972) and human SAMD4A/B (UniProtKB Q9UPU9 and Q5PRF9) were searched using UniProt BLAST with an E value threshold of ten and not gapped [58] . Protein sequences used for the analysis: UniProtKB Q08831 (S. cerevisiae), UniProtKB Q9P6R7 (S. pombe), UniProtKB Q5AI80 (C. albicans), UniProtKB O76699 (C. elegans), UniProtKB Q23972 (D. melanogaster), UniProtKB E7F857, E7FBA1, A0A0R4IUM4 (D. rerio), UniProtKB Q6GLT9, Q5FWP2, A0A1L8GL36 (X. laevis), UniProtKB Q8CBY1, Q80XS6, Q8VIG0 (M. musculus) and UniProtKB Q9UPU9, Q5PRF9, Q8WYQ9 (H. sapiens). MUSCLE v.3.8.31[59] with default parameters was used for multiple sequence alignment of the resulting 17 protein sequences. The phylogenetic tree was reconstructed using PhyML v.3.1/3.0 aLRT60 and visualized using TreeDyn v.198.3 [61] on the Phylogeny.fr platform[62]. The edge and leaves of the reconstructed phylogenetic tree were reordered and rooted manually for visualization.

EXAMPLE 16: RNA Pulldown Assay

The 3' TEG (tetraethylene glycol spacer)-biotinylated RNAs of RNA2.7 stem-loop (SL2.7) and stem-loop mutant (SL2.7 mut) were synthesized (Bioneer Inc.), and the sequences are listed in Table 5.

TABLE 5

| RNA oligos for pulldown mass | |
|---|---|
| SL-2.7 | CACCGCGUUAUCCAUUCCUCGUAGGCUGGUCCUG GGGAACGGGUCGGCGG[Biotin-TEG] |
| SL-2.7 mutant | CACCGCGUUAUCCAUUCCUCGUAGGAUUUUCCUG GGGAACGGGUCGGCGG[Biotin-TEG] |

Streptavidin M-270 beads (Thermo Scientific, 65305) for set 1 and streptavidin magnetic beads (NEB, S1420S) for set 2 were washed twice with pulldown buffer (50 mM Tris (pH 8.0), 150 mM NaCl, 5% glycerol, 1 mM DTT, 100 U/ml RNase inhibitor, 1x Protease inhibitor). Next, 10 $\mu$g of oligonucleotides of SL2.7 or its mutant were conjugated to streptavidin beads in pulldown buffer overnight at 4°C and then washed with pulldown buffer. HEK293T cells from four 150 mm dishes were gathered and then lysed with 1.5 ml pulldown buffer followed by sonication. After centrifugation, supernatant was incubated with beads and washed with wash buffer no. 1 (50 mM Tris (pH 8.0), 300 mM NaCl, 5% glycerol), wash buffer no. 2 (50 mM Tris (pH 8.0), 150 mM NaCl, 5% glycerol, 0.1% triton X-100) and pulldown buffer. For elution, the beads were incubated with 60 $\mu$l of elution buffer (100 mM (pH 7.5), 4% SDS, 100 mM DTT) at 1,600 r.p.m. in thermomixer for 10 min at 65°C. Then, 10 and 50 $\mu$l of eluates were used for western blotting and mass spectrometry, respectively.

EXAMPLE 17: Liquid Chromatography-Tandem Mass Spectrometry (LC-MS/MS) Analysis

The immunoprecipitated protein samples containing SDS were subjected to filter-aided sample preparation digestion. Briefly, protein samples were first reduced and alkylated with denaturing ABC buffer (8 M urea in 50 mM ammonium bicarbonate (ABC)). The alkylated samples were placed on preconditioned 30 kDa MWCO Amicon filter (Millipore, UFC5030) and centrifuged for 30 min at 14,000g. Serial washing was carried out with 200 $\mu$l of 8 M urea or 50 mM ABC buffer and centrifuging for 15 min at 14,000g to remove SDS in the filter unit. Then, protein samples were digested with 2% (w/w) trypsin at 37 °C for overnight. The resulting peptide samples were subject to C18 ziptip clean-up (Millipore, Z720070) and LC-MS/MS analysis. In-house packed long capillary columns (100 cm x 75 $\mu$m inside diameter) and trap columns (3 cm x 150 $\mu$m inside diameter) with 3-$\mu$m Jupiter C18 particles (Phenomenex) were used for peptide separation. A flow rate of 300 nl/min and a linear gradient ranging from 95% solvent A (water with 0.1% formic acid) to 40% of solvent B (acetonitrile with 0.1% formic acid) for 100 min were applied on nanoACQUITY UPLC (Waters) coupled with Orbitrap Fusion Lumos mass spectrometer (Thermo Scientific), which was operated using the following parameters: m/z 300-1,800 of precursor scan range, 1.4 Th of precursor isolation window, 30% of normalized collision energy for higher-energy collisional dissociation (HCD), 30 s of dynamic exclusion duration and a 60,000 or 75,000 resolution at m/z 200 for full MS or MS/MS scan, respectively.

Mass spectrometric raw data files were processed with MaxQuant (v.1.5.3.30) searched using the built-in Andromeda search engine[65] in MaxQuant against the human Uniprot database[58] (v.12/5/2018) at default settings (20 or 6 ppm of precursor ion mass tolerances for initial or main search, respectively, and 0.5 Da for fragment ion masses). Enzyme specificity was set to trypsin/P, and a maximum of two missed cleavages were allowed. Cysteine carbamidomethylation and methionine oxidation were selected as fixed and variable modifications, respectively. A 1% FDR was required at both the protein and the peptide level. The mass spectrometry proteomics data have been deposited to the ProteomeXchange Consortium via the PRIDE[66] partner repository with the dataset identifier PXD018061 and 10.6019/PXD018061. To account for spurious or nonspecific binding to the SL2.7 construct, the results from the "bead only" and the mutant construct were considered as technical background and designed a customized statistical test for protein spectra count enrichment. Let Np be the number of identified spectra count for protein p from the SL2.7 construct, and Mp be the count number from the background construct. For each protein p with Np $\geq$ 1, the statistical significance (or P value) of enrichment was computed:

$$P(X \geq N_p) = \sum_{k=N_p}^{N} B(k; N, \rho)$$

where N=ΣPNP is the total spectra count, ρ=(Mp+1)/ΣP(MP+1) is the background probability, and B(k; n, p) is the binomial distribution of getting k successes in n trials with success probability of P. Finally, P values were adjusted via the Benjamini-Hochberg method. Proteins with an adjusted P < 0.01 when using a technical background ('bead only' or mutant) were considered statistically significant. Statistically significant proteins in either set 1 or set 2 datasets were considered for subsequent protein intensity analysis. To handle missing values, the minimum nonzero intensity value of the dataset was imputed. Protein enrichment score was then calculated as the log2 fold change of the protein intensity of the SL2.7 over the mutant data.

Results

1. HBV and HCMV turn the mixed tailing machinery to their own advantage.

To investigate and characterize viral RNA tailing, TAIL-seq was first applied to two viral infection models: HepG2.2.15 cells incorporated with HBV DNA and primary human foreskin fibroblast (HFF) infected with HCMV. HepG2.2.15 cells were derived from the hepatoblastoma cell line HepG2 and used to study the life cycle of HBV as they sup-port the production and assembly of HBV particles. TAIL-seq data on HepG2.2.15 cells showed exceptionally high guanylation frequency on viral RNAs (FIG. 1a and FIG. 7a). Frequency of mixed tailing (counting G, U and C) is also substantially higher on viral RNAs than cellular RNAs (FIG. 7a). Furthermore, TAIL-seq data on HCMV-infected HFF cells showed high guanylation and mixed tailing frequency on HCMV RNAs (FIG. 1b and FIG. 7b). The modification is attributed mostly to RNA2.7 (FIG. 7c, VRNA2.7), one of the four abundant and conserved long noncoding RNAs of HCMV. Both HBV and HCMV RNAs have substantially longer tails than cellular mRNAs (FIGS. 1c and 1d), indicating that these viral RNAs may undergo slower net deadenylation than cellular mRNAs.

To examine if the viral mixed tailing is mediated by the same enzymes as the host counterparts, the present inventors depleted TENT4A and TENT4B. Guanylation and mixed tailing frequency of HBV mRNAs and HCMV RNA2.7 were reduced in TENT4A- and TENT4B-depleted cells (FIGS. 1e and 1f and FIGS. 7d-7g). Knockdown of TENT4A and TENT4B also led to the shortening of the poly(A) tail of viral RNAs (FIGS. 7h-7j), demonstrating that TENT4 enzymes contribute to the elongation of viral RNA tails. Consistently, HBV mRNAs were downregulated and destabilized after TENT4 depletion (FIGS. 1g and 1h and FIG. 7k), indicating that HBV uses TENT4 to stabilize their mRNAs. Of note, no increase of tail modifications such as oligo-uridylation (FIG. 7l), excluding the possibility of any compensatory mechanism in the absence of TENT4, was found. The highly stable HCMV RNA2.7 did not show a significant change in RNA half-life within the experimental time window (FIG. 7m, P > 0.05; two-sided Student's t-test), suggesting an additional downstream mechanism subsequent to deadenylation, which blocks RNA2.7 decay.

2. HBV RNAs are major substrates of TENT4 in HepG2.2.15 cells.

To elucidate the mechanism of TENT4 recruitment, formaldehyde-mediated crosslinking and immunoprecipitation sequencing (fCLIP-seq) were carried out on HepG2.2.15 cells (FIG. 2). Formaldehyde-based crosslinking captures RNA-protein inter-actions effectively and robustly even when they are transient and structure-dependent. Four libraries were prepared with the input lysates and the immunoprecipitates using normal mouse IgG (NMG), TENT4A antibody or TENT4B antibody. The input and NMG libraries were used as size-matched controls to estimate background. Standardized read coverages across the HBV genome identified substantially enriched peaks from the HBV genome with both TENT4A and TENT4B antibodies (FIG. 2a), indicating the interaction site of TENT4 on HBV RNAs. An independent experiment with TENT4B antibody showed a similar result (FIG. 8a).

HBV RNAs are generated from a circular DNA genome of 3.2 kilobases. Five overlapping viral transcripts share a common 3' end sequence (~700 basepairs) and a single polyadenylation site (FIG. 2a). TENT4-bound reads are enriched in the 3' end region spanning 1,154-1,687 (FIG. 2a). This region corresponds to the post-transcriptional regulatory element (PRE), which is well known to enhance RNA nuclear export and stabilization. PRE of the woodchuck hepatitis virus (WPRE) has been widely used to increase ectopic gene expression and lentiviral gene delivery. While several RNA-binding proteins such as La, PTB, GAPDH and ZC3H18 have been reported to interact with PRE, the functional importance of these interactions remains unclear. In the fCLIP-seq experiment of the present disclosure, the PRE of HBV exhibited the highest enrichment score compared to the other putative peak clusters of the human transcriptome (FIG. 2b). Immunoprecipitation followed by quantitative PCR with reverse transcription (RT-qPCR) also confirmed the interaction between TENT4A, TENT4B and HBV mRNAs (FIG. 8b). Taken together, these results demonstrate that HBV mRNAs are the major substrates of TENT4 in HepG2.2.15 cells and that TENT4 interacts with HBV mRNAs through the PRE region.

3. Stem-loop $\alpha$ of HBV RNAs is necessary for TENT4-dependent tail regulation.

To identify the cis-acting RNA element recognized by TENT4, the present inventors generated reporters with different subregions of PRE in the 3' untranslated region (UTR) (FIG. 3a). The PRE consists of two subelements: PRE$\alpha$ (1154-1351) and PRE$\beta$ (1352-1687) (FIG. 3a). TENT4A and TENT4B knockout (KO) cells were also generated to test TENT4-dependency (FIG. 9a).

Luciferase protein and RNA levels were significantly upregulated in a TENT4-dependent manner only when the reporter was equipped with either the full PRE or PRE$\alpha$ (FIG. 3b and FIG. 9b). Similar results were obtained in human embryonic kidney 293T (HEK293T) cells depleted of TENT4 (FIGS. 9c and 9d), confirming that PRE$\alpha$ is required for TENT4-dependent stabilization. Poly(A) tail length measurement by Hire-PAT assay showed that TENT4 depletion resulted in a shortening of poly(A) tails from the PRE$\alpha$ construct but not from the PRE$\beta$ construct (FIG. 3c).

This result indicates that TENT4 acts directly through tail modification in a PRE$\alpha$-dependent manner. Moreover, in the knockout cells, the PRE and PRE$\alpha$ reporter expressions at both protein and RNA levels were rescued by ectopically expressed TENT4 (FIG. 3d and FIGS. 9e and 9f). The catalytically inactive mutant failed to restore the activity. Thus, the catalytic activity of TENT4 is necessary for the upregulation of mRNA containing PRE$\alpha$.

To examine the mechanistic conservation of PRE, use was made of the woodchuck hepatitis virus (WHV) segment (WPRE) that is homologous to the PRE of HBV (FIG. 3e). Unlike PRE of HBV, WPRE consists of three subelements: WPRE$\gamma$, WPRE$\alpha$ and WPRE$\beta$. WPRE$\alpha$ and WPRE$\beta$ are conserved between WHV and HBV whereas WPRE$\gamma$ is unique and thought to be responsible for the greater activity of WPRE over the PRE of HBV22. All constructs harboring WPRE$\alpha$ (W$\alpha$) showed TENT4-dependent upregulation (FIG. 3f), suggesting that the TENT4-mediated mechanism is conserved in WHV.

The combination of WPRE$\gamma$ (W$\gamma$) and WPRE$\alpha$ (W$\alpha$) resulted in a synergistic effect (FIG. 3f), suggesting that WPRE$\gamma$ could act as an enhancer to TENT4-mediated regulation of WHV.

Further point mutations were introduced to specify the core motif in PRE$\alpha$ (FIG. 3a). PRE$\alpha$ contains a La-binding motif and stem-loop alpha (SL$\alpha$), both of which were reported to be necessary for RNA export and stability. A recent mutagenesis study showed that both the La-binding site and the apical loop sequence of SL$\alpha$ are essential for DHQ-1-mediated HBV gene expression. Of note, the SL$\alpha$ contains a CAGGU pentaloop that essentially adopts the CNGG(N) family loop conformation with a single bulged G residue flanked by A-helical regions (FIG. 3g, left). The structure of SL$\alpha$ is also evolutionarily conserved in WHV (FIG. 3g, right). To test whether these elements are necessary for TENT4-dependent regulation, PRE$\alpha$ mutant reporters (FIG. 3a) that disrupt the apical sequence and shape of SL$\alpha$ (FIG. 3g, arrows) were generated. Mutagenesis of La-binding motif ($\alpha$ mut1) did not affect TENT4-dependency while the SL$\alpha$ mutation ($\alpha$ mut2) and the double mutations ($\alpha$ mut3) abrogated the PRE$\alpha$ function (FIG. 3h). Thus, the stem-loop of PRE$\alpha$ (SL$\alpha$) is an essential element for post-transcriptional regulation by TENT4.

4. HCMV RNA2.7 harbors a similar stem-loop for TENT4- dependent regulation.

In addition to HBV mRNAs, HCMV RNA2.7 (VRNA2.7) also undergoes mixed tailing (FIGS. 1b and 1d and FIG. 7b). An RNA-immunoprecipitation experiment with TENT4A antibody followed by RT-qPCR showed that TENT4A interacts with RNA2.7 that is ectopically expressed in HEK293T cells (FIG. 4a and FIG. 10a), suggesting that no viral factors are required for the interaction between TENT4A and RNA2.7. To identify the functional domain of RNA2.7 responsible for TENT4 interaction, reporters that contain partially overlapping fragments of RNA2.7 (FIG. 4b, FRG1-6) were generated. Protein and RNA levels of the first two reporters (FRG1 and FRG2) were significantly higher in parental cells compared to those in TENT4 KO cells (FIG. 4c and FIG. 10b). Thus, the ciselement is present near the 5' end. Partial deletion of FRG1 revealed that only the 1E fragment (414-513) confers the TENT4-dependent enhancement effect (FIGS. 4d and 4e and FIGS. 10c and 10d). The 1E domain harbors a stem-loop structure (FIG. 4b, lower right) that resembles the CNGG(N) family loop conformation of SL$\alpha$ of PRE$\alpha$ and WPRE$\alpha$ (FIG. 3g). Point mutations to the stem-loop of 1E (1Emut) abrogated the regulation of TENT4 (FIG. 4e and FIG. 10d). A smaller 50-nt truncated fragment that contains the stem-loop (414-463, hereafter referred to as SL2.7) has a similar activity to FRG1 and 1E (FIG. 10e). SL$\alpha$ of HBV PRE and WPRE are near the 3' end of RNA, but SL2.7 of HCMV RNA2.7 is near the 5' end, suggesting that the pentaloop may function in a position-independent manner.

Altogether, the data of present disclosure demonstrate the functional importance of the SL$\alpha$-like structure in TENT4-dependent regulation.

5. Cytoplasmic ZCCHC14 binds to the stem-loop structure and interacts with TENT4.

The CNGGN pentaloop was first characterized as the Smaug recognition element, which binds to the sterile alpha motif (SAM) domain of yeast Vtslp and Drosophila Smaug mediates post-transcriptional repression[32-36]. Based on the structural resemblance, Schwalbe and colleagues had proposed that the HBV SL$\alpha$ pentaloop may serve as a binding site for a protein with the SAM domain. However, TENT4 does not contain a SAM domain[31], and the present inventor's tailing assays with recombinant TENT4 failed to detect a notable affinity to PRE in vitro, indicating an RNA-interacting cofactor.

To find the potential cofactor that recognizes SL$\alpha$ and SL2.7, RNA pulldown was conducted with biotinylated synthetic SL2.7 and HEK293T cell extract (FIG. 5a). The loop mutant was used as a negative control. Mass spectrometry revealed six proteins that are markedly enriched, that is, TENT4A, TENT4B, K0355, SAMD4A, SAMD4B, and ZCCHC14 (FIG. 5a). Particularly, all three human proteins with Smaug-like SAM domains are included in this list: the two homologs of Smaug (SAMD4A and SAMD4B) and ZCCHC14 (FIG. 5b). The specific enrichment of ZCCHC14 was further confirmed by RNA pulldown and western blotting (FIG. 5c). K0355 (or KIAA0355) does not have a SAM domain and it is reported to interact with SAMD4B37, indicating that K0355 may not directly interact with the pentaloop. To identify which SAM domain protein is responsible for TENT4-dependent regulation, reexamination was made of the HBV-producing HepG2.2.15 cells. Knockdown of ZCCHC14 but not that of SAMD4 proteins reduced HBV RNA levels (FIG. 5d and FIG. 11). Consistently, a recent genome-wide CRISPR screen reported ZCCHC14 as an essential host factor for HBV surface antigen production.

To understand the action mechanism of ZCCHC14, TAIL-seq experiments were performed in ZCCHC14-depleted HepG2.2.15 cells. Guanylation frequency of HBV mRNAs was reduced (FIG. 6a), and the poly(A) tail was shortened substantially on ZCCHC14 knockdown (FIG. 6b), demonstrating the critical role of ZCCHC14 in HBV RNA mixed tailing. Consistently, ZCCHC14 depletion led to the reduction of luciferase expression from reporters harboring the CNGGN stem-loop (RNA2.7 1E, PRE$\alpha$, W$\gamma$ + W$\alpha$) (FIG. 6c). In contrast, their respective pentaloop mutants (1E mut, $\alpha$ mut2, W$\gamma$ + W$\alpha$ mut) did not show significant alterations after ZCCHC14 knockdown (P> 0.05; two-sided Student's t-test) (FIG. 6c), indicating that the function of ZCCHC14 depends on the CNGGN motif. The present inventors also carried out Hire-PAT assay to confirm that the poly(A) tail length of reporter mRNAs decreased in ZCCHC14-depleted cells in a CNGGN motifdependent manner (FIGS. 12a-12c).

RNA immunoprecipitation using ZCCHC14 antibody confirmed an interaction between ZCCHC14 and HBV transcripts in HepG2.2.15 cells (FIG. 6d and FIG. 12d) . Moreover, in both HepG2.2.15 and primary HFF cells, ZCCHC14 co-immunoprecipitates with TENT4A and TENT4B regardless of RNase treatment (FIG. 12e), indicating an RNA-independent interaction between ZCCHC14 and TENT4. Last, the present inventors found that ZCCHC14 is mostly localized in the cytoplasm (FIG. 6e) and that ZCCHC14 coimmunoprecipitates with TENT4A and TENT4B specifically using the cytosol fraction of HeLaT cells (FIG. 6f). Collectively, the results of the present disclosure indicate that ZCCHC14 interacts with TENT4 mainly in the cytoplasm to inadvertently protect viral transcripts harboring SL$\alpha$-like elements from deadenylation.

The present inventors revealed the mechanism of targeted mixed tailing in viral gene expression (FIG. 6g). HBV mRNAs and HCMV RNA2.7 contain cis-acting elements with a CNGGN pentaloop that is critical for recruiting ZCCHC14 and TENT4. The mixed tail by TENT4 counteracts the CNOT deadenylase complex, thereby protecting the viral RNAs. It is intriguing that these two distinct viruses (belonging to Hepadnaviridae and Herpesviridae, respectively) with very different life cycles and tissue specificity came up with an identical strategy for the benefit of their gene expression. This convergent evolution and the simplicity of this cis-acting element suggest that this mechanism may be used by other viruses as well.

Other than mixed tailing machinery, TENT4B works as a component of nucleolar TRAMP complex (Trf4-Air1/2-Mtr4 in yeast and TENT4B-ZCCHC7-hMTR4 in human), and is involved in the degradation of aberrant transcripts or the trimming and maturation of small noncoding RNAs in the nucleus[5, 39-43]. The present inventors previously showed that mixed tailing contributes to mRNA stability as a module distinct from TRAMP complex. Given that the knockdown of hMTR4 and ZCCHC7 does not affect the HBV protein and mRNA levels, TENT4 seems to act independently of the TRAMP complex in HBV regulation. Cytoplasmic localization of ZCCHC14 also suggests that the TENT4-ZCCHC14 complex operates in a separate subcellular compartment from the nucleolar TRAMP complex although exclusion is not made of the possibility that a minor fraction of the TENT4-ZCCHC14 complex may act in the nucleus.

Mixed tailing seems to be particularly important for HBV gene expression. Four out of five mRNA species (pgRNA, Precore, PreS1 and PreS2/S except X) contain the SL$\alpha$ stem-loop, and their half-lives are substantially reduced when TENT4 enzymes are depleted. Current treatment options include nucleos(t)ide analogs and interferon alpha, but they are not curative and often ineffective, leaving HBV infection as the major global medical burden causing almost 80 million deaths per year. Functional cure can be achieved by blocking the production of viral proteins, especially viral S antigen (HBsAg) that induces immune evasion. The discovery of viral mixed tailing and the involvement of TENT4 and ZCCHC14 in viral gene expression may provide mechanistic insights for the development of a new class of anti-HBV drugs. To avoid any potential side effects of targeting the TENT4 machinery, it will be important to understand the endogenous functions and action mechanisms of the TENT4 proteins and their partners.

The physical interaction between the CNGGN pentaloop and RNA-binding SAM proteins appears to be a highly conserved and widely used functional module. In fruit fly, Smaug recognizes the hairpin with a CUGGC loop of nanos mRNA to induce timely mRNA degradation, which is essential for the establishment of the anterior-posterior axis. In yeast, Vtslp binds to mRNA with the CNGG(N) pentaloop for targeted mRNA degradation. Vertebrates have three RNA-binding SAM proteins and seem to have expanded the use of this interaction module. SAMD4B, one of the vertebrate orthologs of Smaug, targets nanos1 mRNA and is essential during mammalian neural development. SAMD4A, the other vertebrate ortholog of Smaug, suppresses the translation of pentaloop-containing reporters and is associated with the formation of cytoplasmic foci. Here, the present inventors show that the same mechanism could be used for the opposite purpose via a vertebrate-specific protein ZCCHC14, in particular for the benefit of viral RNAs.

**Claims**

1. A pharmaceutical composition for prevention of viral infection or treatment of viral infection symptoms, the composition comprising an inhibitor against at least one of TENT4A and TENT4B, both responsible for mixed tailing of viral RNA, wherein the inhibitor is siRNA or shRNA inhibiting expression of at least one of TENT4A and TENT4B, or an antibody or an antigen binding fragment thereof inhibiting activity of at least one of TENT4A and TENT4B.

2. The pharmaceutical composition of claim 1, wherein TENT4A and TENT4B are derived from a subject which is at risk of viral infection or has been infected with virus.

3. The pharmaceutical composition of claim 1, wherein the virus is a virus which undergoes RNA tailing induced by at least one of TENT4A and TENT4B.

4. The pharmaceutical composition of claim 3, wherein the virus is a virus belonging to the family Hepadnaviridae or Herpesviridae.

5. The pharmaceutical composition of claim 4, wherein the virus belonging to the family Hepadnaviridae is any one selected from the group consisting of hepatitis B virus, ground squirrel hepatitis B virus, woodchuck hepatitis B virus, duck hepatitis B virus, and heron hepatitis B virus.

6. The pharmaceutical composition of claim 4, wherein the virus belonging to the family Herpesviridae is any one selected from the group consisting of Iltovirus, Mardivirus, Scutavirus, Simplexvirus, Varicellovirus, Cytomegalovirus, Muromegalovirus, Proboscivirus, Roseolovirus, Lymphocryptovirus, Macavirus, Percavirus, and Rhadinovirus.

7. A method for screening a pharmaceutical composition for prevention of viral infection or treatment of viral infection symptoms, the method comprising the steps of:

   (a) transfecting a virus into host cells;
   (b) treating the host cells with a drug candidate material; and
   (c) analyzing an RNA tailing level of a test group treated with the drug candidate material and comparing the RNA tailing level with that of a control treated without the drug candidate material,
   wherein the drug candidate material is selected as an effective candidate when the RNA tailing level of the test group is relatively reduced compared to the non-treated control.

8. A method for preparing a virus-resistant cell, the method comprising a step of knocking out expression of at least one of TANT4A and TENT4B in a cell isolated from a living body.

9. A method for stabilizing an RNA sequence, the method comprising a step of inserting a stem-loop sequence including a pentaloop structure consisting of the amino acid sequence represented by the following general formula into a target RNA sequence:

   [General Formula]
   5'-CNGGN-3' ,
   wherein N's are each independently selected from adenosine (A) and uracil (U).

**FIG. 1a**

**FIG. 1b**

**FIG. 1c**

c

HepG2.2.15

Cellular (73 nt)
HBV (117 nt)

Density (%)

Poly(A) tail length (nt)

**FIG. 1d**

d HCMV-infected HFF

Cellular (62 nt)
HCMV (91 nt)
*VRNA*2.7 (156 nt)

**FIG. 1e**

**FIG. 1f**

f

HCMV   VRNA2.7

**FIG. 1g**

**FIG. 1h**

**FIG. 2a**

**FIG. 2b**

**FIG. 3a**

a

Sub-PRE region constructs

**FIG. 3b**

b

**FIG. 3c**

**FIG. 3d**

**FIG. 3e**

Sub-WPRE region constructs

**FIG. 3f**

**FIG. 3g**

Stem-loop structure

PRE
stem-loop α
(1,297–1,320)

WPRE
stem-loop
(1,426–1,445)

**FIG. 3h**

**FIG. 4a**

**FIG. 4b**

b

Sub-RNA2.7 region constructs

HCMV RNA2.7 stem-loop (429–451)

**FIG. 4c**

**FIG. 4d**

**FIG. 4e**

**FIG. 5a**

**FIG. 5b**

**FIG. 5c**

**FIG. 5d**

**FIG. 6a**

**a**

HBV

Guanylated tail (%)

- Internal
- Penultimate
- Terminal

siCont  siZCCHC14

**FIG. 6b**

b     HBV

Legend:
- siCont (114.5 nt)
- siZCCHC14 (75 nt)

Density (%) vs Poly(A) tail length (nt)

**FIG. 6c**

**FIG. 6d**

**FIG. 6e**

**FIG. 6f**

**FIG. 6g**

FIG. 7a

a

HepG2.2.15

FIG. 7b

FIG. 7c

## C

### HCMV-infected HFF

Cellular      HCMV

VRNA2.7

Guanylated read count (K)

Poly(A) read count (K)

**FIG. 7d**

**FIG. 7e**

FIG. 7f

f

HCMV-infected HFF

TENT4A · · · TENT4B

**FIG. 7g**

g

HCMV RNA2.7

**FIG. 7h**

# h

## HBV

Legend:
- siCont (117 nt)
- siTENT4 (59.5 nt)

Y-axis: Density (%)
X-axis: Poly(A) tail length (nt)

**FIG. 7i**

i

HCMV

Density (%)

1.5

1.0

0.5

0

☐ siCont (91 nt)
■ siTENT4 (67 nt)

0    50   100  150  200  250

Poly(A) tail length (nt)

**FIG. 7j**

**FIG. 7k**

**FIG. 7I**

FIG. 7m

**FIG. 8a**

FIG. 8b

FIG. 9a

EP 3 984 558 A1

**FIG. 9b**

**FIG. 9c**

# C

## HEK293T

**FIG. 9d**

**FIG. 9e**

FIG. 9f

**FIG. 10a**

**FIG. 10b**

**FIG. 10c**

**FIG. 10d**

**FIG. 10e**

FIG. 11

EP 3 984 558 A1

FIG. 12a

**FIG. 12b**

Stem-loop structure

WPRE
stem-loop
(1325-1341)

WPRE
stem-loop
(1426-1445)

**FIG. 12c**

FIG. 12d

**FIG. 12e**

**FIG. 13**

<div style="text-align:center">

**INTERNATIONAL SEARCH REPORT**

</div>

| International application No. |
|---|
| PCT/KR2020/007100 |

| A. CLASSIFICATION OF SUBJECT MATTER |
|---|
| *A61K 48/00(2006.01)i, A61K 31/713(2006.01)i, A61K 31/7105(2006.01)i, A61P 31/20(2006.01)i, A61P 31/22(2006.01)i, C12Q 1/70(2006.01)i, C12N 15/113(2010.01)i*  |
| According to International Patent Classification (IPC) or to both national classification and IPC |

| B. FIELDS SEARCHED |
|---|
| Minimum documentation searched (classification system followed by classification symbols) |
| A61K 48/00; A61K 31/712; A61K 31/7125; G01N 33/50; A61K 31/713; A61K 31/7105; A61P 31/20; A61P 31/22; C12Q 1/70; C12N 15/113 |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & Keywords: virus, RNA, tailing, TENT4A, TENT4B, inhibitor, stabilization, CNGGN |

| C. DOCUMENTS CONSIDERED TO BE RELEVANT |
|---|

| Category* | Citation of document, with indication, where appropriate, of the relevant passages | Relevant to claim No. |
|---|---|---|
| X | KR 10-2019-0019164 A (F. HOFFMANN-LA ROCHE AG.) 26 February 2019<br>See claims 1 and 7. | 1-6,8 |
| X | LIM, J. et al. Mixed tailing by TENT4A and TENT4B shields mRNA from rapid deadenylation. Science. 2018, vol. 361, pages 701-704<br>See abstract; and pages 2 and 3. | 7 |
| Y | CHEN, L. et al. Global regulation of mRNA translation and stability in the early Drosophila embryo by the Smaug RNA-binding protein. Genome Biology. 2014, vol. 15, document no. R4, pages 1-21<br>See abstract; and pages 6 and 7. | 9 |
| Y | EMORY, S. A. et al. A 5'-terminal stem-loop structure can stabilize mRNA in Escherichia coli. GENES & DEVELOPMENT. 1992, vol. 6, pages 135-148<br>See abstract. | 9 |
| X | WO 2019-076842 A1 (F. HOFFMANN-LA ROCHE AG. et al.) 25 April 2019<br>See claim 1; and pages 5 and 6. | 1-6,8 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 SEPTEMBER 2020 (10.09.2020) | **11 SEPTEMBER 2020 (11.09.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2020/007100**

| Patent document cited in search report | Publication date | Patent family member | Publication date |
|---|---|---|---|
| KR 10-2019-0019164 A | 26/02/2019 | AU 2017-285350 A1 | 20/12/2018 |
| | | AU 2017-285351 A1 | 13/12/2018 |
| | | BR 112018075682 A2 | 02/04/2019 |
| | | BR 112018075960 A2 | 01/10/2019 |
| | | CA 3027305 A1 | 21/12/2017 |
| | | CA 3027811 A1 | 21/12/2017 |
| | | CL 2018003561 A1 | 15/03/2019 |
| | | CN 109328237 A | 12/02/2019 |
| | | CN 109414448 A | 01/03/2019 |
| | | CR 20180590 A | 05/03/2019 |
| | | EP 3472346 A1 | 24/04/2019 |
| | | EP 3472362 A1 | 24/04/2019 |
| | | JP 2019-518037 A | 27/06/2019 |
| | | JP 2019-523649 A | 29/08/2019 |
| | | KR 10-2019-0018515 A | 22/02/2019 |
| | | MX 2018015516 A | 18/03/2019 |
| | | MX 2018015521 A | 18/03/2019 |
| | | MX 367541 B | 22/08/2019 |
| | | MX 367543 B | 22/08/2019 |
| | | PE 20190443 A1 | 29/03/2019 |
| | | RU 2019100094 A | 17/07/2020 |
| | | SG 11201810594 A | 28/12/2018 |
| | | US 2019-0194768 A1 | 27/06/2019 |
| | | US 2019-0216846 A1 | 18/07/2019 |
| | | WO 2017-216390 A1 | 21/12/2017 |
| | | WO 2017-216391 A1 | 21/12/2017 |
| WO 2019-076842 A1 | 25/04/2019 | AU 2018-350693 A1 | 12/03/2020 |
| | | CA 3072314 A1 | 25/04/2019 |
| | | EP 3645723 A1 | 06/05/2020 |
| | | KR 10-2020-0030594 A | 20/03/2020 |
| | | KR 10-2020-0094230 A | 06/08/2020 |
| | | TW 201923080 A | 16/06/2019 |
| | | US 2019-0111073 A1 | 18/04/2019 |
| | | US 2020-0147123 A1 | 14/05/2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   KR 1020190064129

**Non-patent literature cited in the description**

*   Hepatitis B virus biology. *Microbiol Mol Biol Rev.,* 2000, vol. 64, 51-68
*   Hepatitis B virus infection natural history and clinical consequences. *N Engl J Med.,* 2004, vol. 350, 1118-1129
*   Hepatitis B virus e antigen the dangerous end game of hepatitis B virus. *N Engl J Med.,* 2002, vol. 347, 208-210